# EUROPEAN PATENT APPLICATION

(11) **EP 3 912 628 A1**
(43) Date of publication of application: **24.11.2021**
(21) Application number: 20305537.1
(22) Date of filing: 20.05.2020
(51) Int. Cl.: A61K 31/706, A61K 31/7076

(54) **NUCLEOSIDE ANALOGUES TO INHIBIT THE MAIN PROTEASE OF A CORONAVIRUS**

(71) Applicant: Institut de Recherche en Semiochimie et Ethologie Appliquée, 84400 Apt (FR)
(72) Inventor: PAGEAT, Patrick, 84400 APT (FR); LORMIER, Anh Tuan, 13800 ISTRES (FR)
(74) Representative: Ernest Gutmann - Yves Plasseraud S.A.S.

(57) **Abstract**

The present invention is directed to nucleoside analogues to inhibit the main protease of a coronavirus. In particular the present invention relates to a composition comprising (i) a compound selected from the group consisting of clitocine, a pharmacophore for clitocine, tautomer, mesomer, racemate, enantiomer, diastereomer, or mixture thereof, or an acceptable salt thereof, and (ii) a pharmaceutically acceptable vehicle, a carrier, an excipient or a diluent, for use in the prevention and/or the treatment of an infection by a virus from the *Coronaviridae* family or an illness related to such infection, in a host, in particular a mammalian host.

## Description

The present invention is directed to nucleoside analogues to inhibit the main protease of a coronavirus. In particular the present invention relates to a composition comprising (i) a compound selected from the group consisting of clitocine, a pharmacophore for clitocine, tautomer, mesomer, racemate, enantiomer, diastereomer, or mixture thereof, or an acceptable salt thereof, and (ii) a pharmaceutically acceptable vehicle, a carrier, an excipient or a diluent, for use in the prevention and/or the treatment of an infection by a virus from the *Coronaviridae* family or an illness related to such infection, in a host, in particular a mammalian host.

Coronaviruses are enveloped, positive-sense, single-stranded RNA viruses that belong to the *Coronaviridae* family. Coronaviruses have a characteristic morphology with a "club-shaped" surface. Their genome encodes four or five structural proteins [a spike (S) protein, an envelope (E) protein, a membrane (M) protein, a nucleocapsid (N) protein, and sometimes a hemagglutinin esterase (HE) protein], non-structural proteins that are released from polyproteins cleaved by the virus proteases, and further comprises additional open reading frames coding for proteins of unknown functions. Coronaviruses can infect humans and a large variety of animals. Examples of coronaviruses include severe acute respiratory syndrome coronavirus (SARS-CoV), severe acute respiratory syndrome coronavirus 2 (SARS-CoV-2), feline coronavirus (FCoV), porcine respiratory coronavirus (PRCV), porcine transmissible gastroenteritis virus (TGEV), porcine epidemic diarrhea virus (PEDV), bovine coronavirus (BCV), bovine coronavirus (BCoV), canine coronavirus (CCoV), avian coronavirus (IBV), and middle east respiratory syndrome-coronavirus (MERS-CoV).

Severe acute respiratory syndrome coronavirus (SARS-CoV-1) is a strain of virus that emerged in 2002-2004 as an outbreak in Asia and was observed to cause severe acute respiratory syndrome (SARS) or at least often caused a severe disease with people showing systemic and respiratory symptoms. Currently there is no vaccine or effective treatment for SARS-CoV-1.

The newly 2019 identified coronavirus was first named 2019-nCoV before being officially named severe acute respiratory syndrome coronavirus 2 (SARS-CoV-2). In the coronavirus family, SARS-CoV-2 is a SARS-like virus but is different from SARS-CoV-1 identified in 2002. SARS-CoV-2 has been characterized to date as a single-stranded RNA betacoronavirus with a genome of 30kb encoding as many as 14 open reading frames (ORFs) (Gordon D.E. et al, Nature, 2020 doi.org/10.1038/s41586-020-2286-9)*.* A polyprotein is encoded by the ORF1a/ORF1ab and is auto-proteolitically cleaved into 16 non-structural proteins (Nsp1-16) that form the replicase/transcriptase complex (RTC). The RTC contains enzymes including the Nsp3 [papain-like protease], Nsp5 (main protease, also named Mpro), the Nsp7-Nsp8 primase complex, the primary RNA-dependent RNA polymerase (Nsp12), a helicase/triphosphatase (Nsp13), an exoribonuclease (Nsp14), an endonuclease (Nsp15) and N7- and 2'O-methyltransferases (Nsp10/Nsp16)] (Chan J.F.W. et al. Emerg. Microbes Infect., 2020, 9, 221-236). Additionally the genome expresses 13 ORFs at its end that include four structural proteins: Spike (S), Envelope (E), Membrane (M) and Nucleocapsid (N) together with 9 putative accessory factors (FehrA.R. & Perlman, Methods. Mol. Biol., 2015, 1282, 1-23). In the RNA that encodes the predicted Nsp1-16 proteins and the structure proteins, SARS-CoV-2 is very similar to SARS-CoV-1 identified in 2002. By contrast the two viruses differ in their 3'ORFs, SARS-CoV-2 exhibiting an ORF3b and an ORF10 with very limited homology to SARS-CoV-1. This new strain of SARS virus causes an infectious respiratory disease called coronavirus disease 2019 (COVID-19). People infected with COVID-19 can be asymptomatic or show symptoms of mild, moderate or severe intensity which can be fatal. On March 11, 2020, the World Health Organization (WHO) characterized COVID-19 as a pandemic affecting both industrialized and developing countries and declared COVID-19 as a Public Health Emergency of International Concern. In this context, experts gathered at WHO agreed that the development of a vaccine and/or a treatment for COVID-19, which do not exist yet, is a major priority around the world.

Feline coronavirus (FCoV) is a coronavirus that infects cats worldwide. Two forms of FCoV are found in nature: feline enteric coronavirus (FECV) and feline infectious peritonitis virus (FIPV) that causes the disease feline infectious peritonitis (FIP). There is currently no effective treatment for FIP.

Transmissible gastroenteritis virus or porcine transmissible gastroenteritis virus (TGEV) is a coronavirus that infects pigs and causes the disease transmissible gastroenteritis (TGE). Symptoms include severe diarrhea, vomiting and rapid dehydration. Porcine respiratory coronavirus (PRCV) is a closely related virus, which is derived from TGEV by deletion of the S-gene. Canine coronavirus and FIPV are also related to TGEV. There is no specific treatment for TGE.

Porcine epidemic diarrhea virus (PEDV) is an alpha coronavirus that infects pigs and causes the disease porcine epidemic diarrhea (PED) with a high mortality in neonatal pigs, resulting in a severe agricultural loss. Symptoms include severe diarrhea, vomiting and dehydration. There is a major outbreak in swine producing countries, in particular in Europe and Asia. There is currently no effective treatment for PED.

Bovine coronavirus (BCoV) is a coronavirus that infects a bovine animal and causes enteric and respiratory disease. Symptoms include diarrhea and respiratory illnesses. BCoV is a biologically significant respiratory pathogen in cattle (Ellis, J. The Canadian veterinary journal, 2019, 60(2), 147-152). There is currently no effective treatment.

In order to find the best drug target candidate to prevent or treat coronavirus infections, it is necessary to fully understand the characteristics of said infections. The inventors have considered various coronaviruses that have been known to infect animals for years for which no treatment is yet available and have designed an approach using the 3D-structure of viral proteases in different strains with a view to identify molecules that could target structures shared in these proteases for ligand-protease interactions, e.g. efficient binding. Targeting viral proteases is indeed one of the routes to provide treatments against COVID-19 and more broadly against coronavirus infection. Accordingly viral protease inhibition would prevent cleavage of the virus polyproteins and would thus hamper virus replication and assembly. The invention accordingly originates from molecular docking studies (virtual screening studies) and binding site analysis of protease of several coronaviruses that enabled to identify binding capability for molecules capable of inhibiting virus proteases. Besides SARS-CoV-2 study, data were obtained from other *Coronaviridae* to enable determining candidate ligands and their binding affinity with a view to define candidate molecules for infection *treatment.*

The coronavirus main protease (Mpro) plays a vital role in viral replication through the proteolytic processing of the polyproteins and is thus an attractive drug target. Its X-ray crystal structure has been recently published (X. Liu et al, PDB 2020, DOI 10.210/pdb6lu7/pdb*).* The coronavirus Mpro is known to cleave at 11 positions, *i.e*. 11 cleavage sites. For example, the SARS-CoV-1 Mpro cleaves the replicase polyproteins, pp1a and pp1b, at 11 specific positions, using core sequences in the polyprotein substrate to determine cleavage sites (T. Muramatsu, et al., Proc. Natl. Acad. Sci. USA 2016, 113, 12997-13002*).* The SARS-CoV-2 Mpro has 96% identity with the SARS-CoV-1 Mpro.

In the present invention, the inventors studied the molecular interactions of a high-resolution experimental structure of the Mpro of SARS-CoV-1, SARS-CoV-2, FIPV, TGEV and PEDV, or of the HE of BCoV with nucleoside analogues using docking analysis. In particular the inventors surprisingly found that clitocine and pharmacophores for clitocine had molecular interactions with an active site, *i.e*. a substrate binding site, in Mpro or HE of viruses involved in SARS, COVID-19, FIP, TGE, PED, and enteric and respiratory disease. Thus clitocine and pharmacophores for clitocine may inhibit the Mpro or HE of a coronavirus and be regarded as drug candidate for use in the treatment and/or the prevention of coronavirus infections or illness related to infection, in particular respiratory illness.

Clitocine is a natural amino nucleoside, especially an adenosine nucleoside analogue, that has been first isolated from the mushroom *Clitocybe inversa (*Kubo et al., Tet. Lett., 1986, 27: 4277). Clitocine has been reported as a potential novel therapeutic agent to overcome drug resistance in cancer therapy (Sun et al., Apoptosis, 2014, 19(5), 871-882) and has been shown to have growth inhibitory activity against lung, colon and gastric human cancer cells (Vaz et al., Food and Chemical Toxicology, 2010, 48(10), 2881-2884). EP3067053 and US16/342640 patent applications disclose the use of clitocine as a therapeutic compound for diseases associated with a nonsense mutation.

The invention relates to a compound selected from the group consisting of clitocine, a pharmacophore for clitocine, tautomer, mesomer, racemate, enantiomer, diastereomer, or mixture thereof, or an acceptable salt thereof, for use in the prevention and/or the treatment of an infection by a virus from the *Coronaviridae* family or an illness related to such infection, in a host, in particular a mammalian host.

The invention also relates to a composition, in particular a pharmaceutical composition, comprising (i) clitocine, a pharmacophore for clitocine, tautomer, mesomer, racemate, enantiomer, diastereomer, or mixture thereof, or an acceptable salt thereof, and (ii) a pharmaceutically acceptable vehicle, a carrier, an excipient or a diluent, for use in the prevention and/or the treatment of an infection by a virus from the *Coronaviridae* family, i.e. a coronavirus infection, or an illness related to such infection, in a host, in particular a mammalian host.

As used herein, the term *"nucleoside analogues"* refers to nucleosides containing a nucleic acid analogue and a sugar.

Clitocine (CAS Registry number 105798-74-1; PubChem CID129111; CHEBI:205898) as used herein is also known as (2R,3R,4S,5R)-2-[(6-amino-5-nitropyrimidin-4-yl)amino]-5-(hydroxymethyl)oxolane-3,4-diol (IUPAC name); or 6-amino-5-nitro-4-(ribofuranosylamino)pyrimidine; or beta-D-ribofuranosylamine, N-(6-amino-5-nitro-4-pyrimidinyl); or 2-(6-amino-5-nitro-pyrimidin-4-ylamino)-5-hydroxymethyl-tetrahydro-furan-3,4-diol; or (2R,3R,4S,5R)-2-(6-amino-5-nitro-pyrimidin-4-ylamino)-5-hydroxymethyl-tetrahydro-furan-3,4-diol. Clitocine has the molecular formula of C₉H₁₃N₅O₆, which is represented by the general formula (I) below:

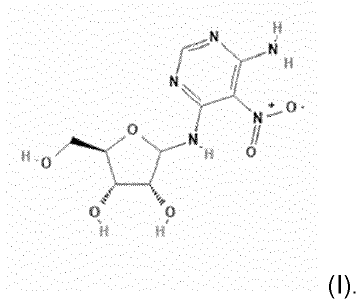

Clitocine as used herein can be the alpha-anomer of clitocine, the beta-anomer of clitocine, or a mixture of alpha and beta anomers. Preferably, clitocine is the beta anomer represented by the formula (II) below:

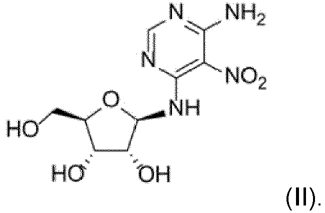

Clitocine may be obtained by synthesis according to published procedure (Moss R.J. et al, 1988, J Med Chem 31: 786-790*; Korean patent application* KR20060102602). Clitocine is commercially available and can be purchased for example from Muse Chem (USA), MedKoo Biosciences, Inc. (USA), Angene and Interchim.

As used herein, the term "a *pharmacophore for clitocine"* refers to a compound comprising the functional properties of clitocine of binding to a protease, in particular Mpro, or HE of a coronavirus, especially of SARS-CoV-1, SARS-CoV-2, FIPV, TGEV, PEDV or BCoV, and optionally structural features of clitocine. In a particular embodiment, the pharmacophore for clitocine according to the invention exhibits the same biological activity as clitocine as inhibitor of the Mpro or HE activity. In another particular embodiment, the pharmacophore for clitocine is active in preventing and/or treating an infection by a virus from the *Coronaviridae* family, *i.e*. a coronavirus infection, or an illness related to such infection, in a host, in particular a mammalian host, especially a human host. In a particular embodiment of the invention, the pharmacophore for clitocine encompasses clitocine derivatives or an adenosine analogue whose pharmacophore features are similar to the pharmacophore features of clitocine. As used herein, the term *"clitocine derivatives"* refers to compounds that are derived from clitocine by a chemical reaction, i.e. by substitution of one or more substituents such as, for example, halogen, alkyl, alkoxy, aryl, heteroaryl, haloalkyl, haloalkoxy, alkoxycarbonyl, alkanoyl, aroyl, formyl, nitrile, nitro, amido, alkylthio, alkylsulfinyl, alkylsulfonyl, arylthio, arylsulfinyl, arylsulfonyl, amino, alkylamino, arylamino, dialkylamino and diarylamino.

A molecule, in particular a pharmacophore for clitocine is considered to be an inhibitor of Mpro or HE of a coronavirus when it prevents or lessens the activity of Mpro or HE on the maturation of the coronavirus, *in vitro* or *in vivo,* in particular the activity of Mpro of SARS-CoV-1, SARS-CoV-2, FIPV, TGEV, PEDV or the activity of HE of BCoV. In a particular embodiment, the activity of the inhibitor may be determined by measurement of the IC50 values that should preferably be lower than 50mM, advantageously lower than 10mM.

Using an *in silico* approach, the inventors evaluated the activity of the inhibitor by estimating the inhibition constant (Ki) value in molecular docking. The inhibition constant values were virtually calculating the chemical activity interaction towards the protein. All the docking results showed values ranging from a minimum of around ∼3.81 µM to a maximum of around 50 µM.

As used herein, the term *"tautomer"* refers to structural isomers differing only in the positions of hydrogen atoms and electrons. Examples of tautomers include, but are not limited to, ketone-enol, enamine-imine, amide-imidic acid, lactam-lactim, nitroso-oxime, ketene-ynol, amino acid, or phosphite-phosphonate.

As used herein, the term *"mesomer"* or "*meso compound"* refers to a stereoisomer that has two or more chiral centers but is optically inactive.

As used herein, the term *"racemate"* or *"racemic mixtures"* refers to a mixture of two enantiomers in equal proportions.

As used herein, the term *"enantiomer"* refers stereoisomers that are mirror images, *i.e.* mirror image isomers.

As used herein, the term *"diastereomer"* refers to isomers of compounds with more than one chiral center that are not mirror images of one another.

As used herein, the term *"an acceptable salt thereof"* refers to any non-toxic salts of a compound of the invention that do not interfere with the activity of the composition.

As used herein, the term *"pharmaceutically acceptable vehicle"* encompasses any substance that does not interfere with the activity of the composition. A vehicle is any substance or combination of substances physiologically appropriate for its use in a composition in contact with a host, in particular a mammalian host, preferably a human host, and thus non-toxic. Pharmaceutically acceptable vehicles are well known in the art.

As used herein, the term *"carrier"* encompasses any standard pharmaceutical carriers such as phosphate buffered saline solutions, water, emulsions, tablets and capsules.

As used herein, the term *"excipient"* encompasses any pharmaceutically acceptable excipients such as binding agents, fillers, lubricants, disintegrants and wetting agents.

As used herein, the term *"diluent"* encompasses an inert agent that is designed to increase the weight of the composition.

In a particular embodiment of the invention, said infection or disease related thereto is selected from the group consisting of SARS, COVID-19, FIP, TGE, PED, enteric and respiratory disease, and MERS, preferably is selected from the group consisting of SARS, COVID-19, FIP, TGE, PED, and enteric and respiratory disease.

In another particular embodiment of the invention, the virus from the *Coronaviridae* family is selected from the group consisting of SARS-CoV-1, SARS-CoV-2, FIPV, TGEV, PEDV, BCoV, FCoV, PRCV, BCV, CCoV and MERS-CoV. Preferably, the virus from the *Coronaviridae* family is selected from the group consisting of SARS-CoV-1, SARS-CoV-2, FIPV, TGEV, PEDV and BCoV.

In a preferred embodiment of the invention, the composition comprises clitocine.

In another preferred embodiment of the invention, the compound is selected from the group consisting of compounds 2-90, preferably from the group consisting of compounds 2-76, even more preferably from the group consisting of compounds 2-43 as disclosed herein. In another more preferred embodiment of the invention, the compound is selected from the group consisting of compounds 2-10, 11 and 77-90. In another more preferred embodiment of the invention, the compound is selected from the group consisting of compounds 2-10.

In a preferred embodiment of the invention, the mammalian host is a human host.

In another preferred embodiment of the invention, the mammalian host is selected from the group consisting of a pig, a bovine animal, a horse, a cat, a dog, a rabbit, a rodent, a bird and a bat, preferably is a pig, a bovine animal or a cat.

When using a compound that is a pharmacophore for clitocine, the compound may be selected in the particular groups of compounds identified herein and may advantageously be further selected for optimized binding properties toward the Mpro or HE of the specific coronavirus that one intends to treat in the host in need of such treatment.

In a particular embodiment of the invention, the composition is for use in association with another therapeutic agent, in particular an antibiotic, in the prevention and/or the treatment of an infection by a virus from the *Coronaviridae* family or an illness related to such infection according to the invention.

The present invention also relates to a method to prevent and/or treat an infection by a virus from the *Coronaviridae* family, i.e. a coronavirus infection, or a disease related to such infection, in a host, in particular a mammalian host, preferably a human host, or an animal host known to be sensible to infection by especially one of the herein cited coronaviruses, comprising administering a pharmaceutically effective quantity of the composition according to the invention.

As used herein, the term *"to prevent"* refers to a method by which the coronavirus infection is obstructed or delayed.

As used herein, the term *"to treat"* refers to a method by which the symptoms of the coronavirus infection are either alleviated, i.e. decrease of the coronavirus infection in the host (especially of the measured virus load) or improvement of the clinical condition of the patient, or completely eliminated. In particular the composition and the method of the invention are used to treat the respiratory disease associated with coronavirus infection, in particular with SARS or COVID-19 in a human host.

As used herein, the term "*a pharmaceutically effective quantity*" refers to an amount which is sufficient to prevent and/or treat a patient at risk for developing or diagnosed with a coronavirus infection, thus producing the desired therapeutic effect.

The present invention also relates to a method to inhibit the main protease (Mpro) or a hemagglutinin esterase (HE) of a virus from the *Coronaviridae* family, i.e. a coronavirus, in a host, in particular a mammalian host, preferably a human host, comprising administering a pharmaceutically effective quantity of the composition according to the invention.

In a preferred embodiment of the invention, the Mpro of a virus from the *Coronaviridae* family has an amino acid sequence selected from the group consisting of SEQ ID NO: 1, SEQ ID NO: 2, SEQ ID NO: 3, SEQ ID NO: 4, SEQ ID NO: 5 and SEQ ID NO: 6, and the BCoV-HE has the amino acid sequence of SEQ ID NO: 7.

As used herein, the term *"to inhibit the main protease (Mpro) of a coronavirus"* refers to preventing or lessening the activity of Mpro on the maturation of the coronavirus, in particular with SARS or COVID-19. The activity of the inhibitor may be determined by measurement of the IC50 values that should preferably be lower than 50mM.

The inventors collected compounds against CID_129111 in PubChem database based on Tanimoto similarity coefficient analysis. The compounds (.sdf format) were also collected according to the CID number and the structures were converted into 3D coordinates using Open Bable software. The inventors observed the pharmacophore model of clitocine and identified the chemical features of the compounds.

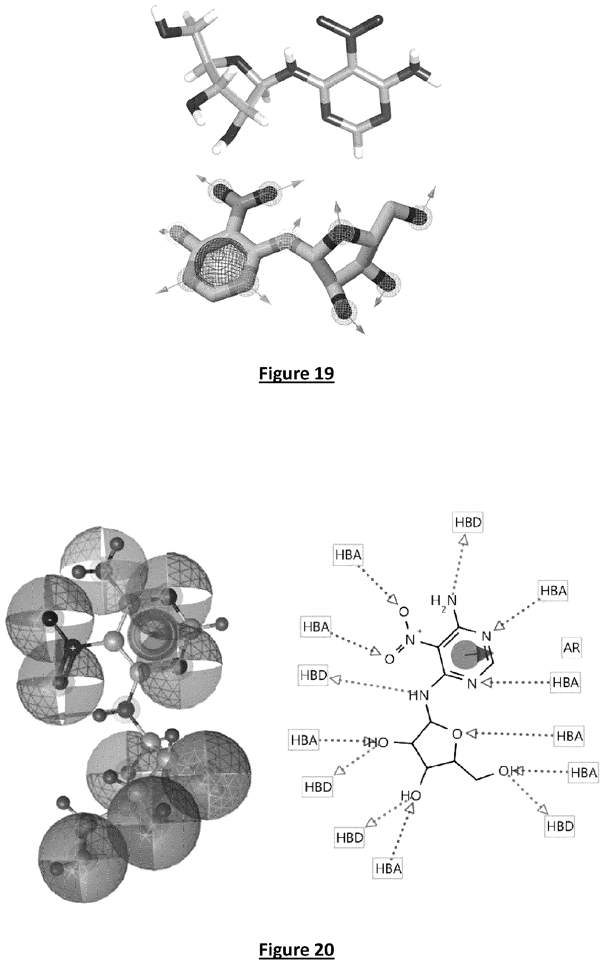

The 3D structure of clitocine (CID 129111) is represented in **Figure 19****,** and its pharmacophore structure is represented in **Figure 20****.**

First of all, the inventors carried out a fingerprint Tanimoto-based 2-dimensional similarity search (Tanimoto threshold = 100%) of clitocine (compound CID129111) and identified 10 compounds **(compounds 1-10)** as disclosed in **Table 1** including clitocine as compound 1.

**Table 1. List of identified compounds 1-10 and their pharmacophore features (HBA = Hydrogen Bond Acceptor; HBD = Hydrogen Bond Donor; AR = Aromatic Ring).**

| **Compound** | **PubChem CID** | **Compound name** | **IUPAC name** | **Pharmacophore features** |
|---|---|---|---|---|
| 1 | 129111 | Clitocine | (2R,3R,4S,5R)-2-[(6-amino-5-nitropyrimidin-4-yl)amino]-5-(hydroxymethyl)oxolan e-3,4-diol | 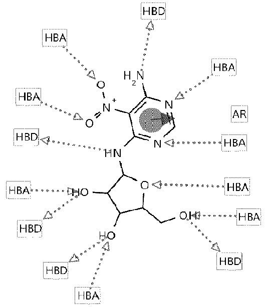 |
| 2 | 11644921 | 2-[(6-Amino-5-nitropyrimidin-4-yl)amino]-5-(hydroxymethyl)oxolane-3,4-diol | 2-[(6-amino-5-nitropyrimidin-4-yl)amino]-5-(hydroxymethyl)oxolane-3,4-diol | 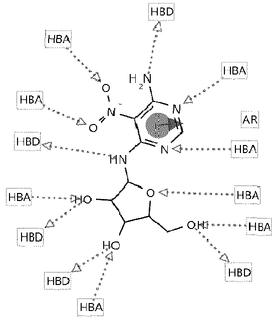 |
| 3 | 13965719 | (3R,4S,5R)-2-[(6-Amino-5-nitropyrimidin-4-yl)amino]-5-(hydroxymethyl)oxolane-3,4-diol | (3R,4S,5R)-2-[(6-amino-5-nitropyrimidin-4-yl)amino]-5-(hydroxymethyl)oxolane-3,4-diol | 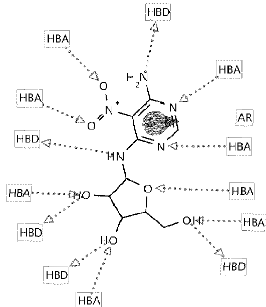 |
| 4 | 13965721 | (2R,3R,4R,5R)-2-[(6-Amino-5-nitropyrimidin-4-yl)amino]-5-(hydroxymethyl)oxolane-3,4-diol | (2R,3R,4R,5R)-2-[(6-amino-5-nitropyrimidin-4-yl)amino]-5-(hydroxymethyl)oxolane-3,4-diol | 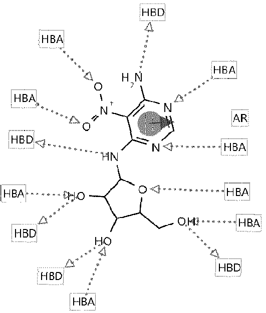 |
| 5 | 58996359 | (2S,3S,4R,5S)-2-[(6-Amino-5-nitropyrimidin-4-yl)amino]-5-(hydroxymethyl)oxolane-3,4-diol | (2S,3S,4R,5S)-2-[(6-amino-5-nitropyrimidin-4-yl)amino]-5-(hydroxymethyl)oxolane-3,4-diol | 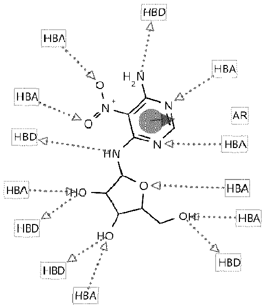 |
| 6 | 60139991 | (2S,3R,4S,5R)-2-[(6-Amino-5-nitropyrimidin-4-yl)amino]-5-(hydroxymethyl)oxolane-3,4-diol | (2S,3R,4S,5R)-2-[(6-amino-5-nitropyrimidin-4-yl)amino]-5-(hydroxymethyl)oxolane-3,4-diol | 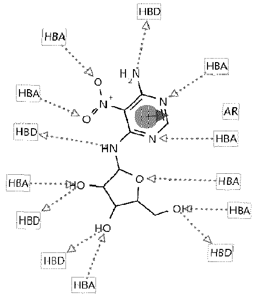 |
| 7 | 71136912 | (2R,5R)-2-[(6-Amino-5-nitropyrimidin-4-yl)amino]-5-(hydroxymethyl)oxolane-3,4-diol | (2R,5R)-2-[(6-amino-5-nitropyrimidin-4-yl)amino]-5-(hydroxymethyl)oxolane-3,4-diol | 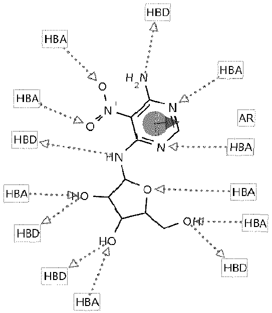 |
| 8 | 90294771 | (2R,3S,5R)-2-[(6-Amino-5-nitropyrimidin-4-yl)amino]-5-(hydroxymethyl)oxolane-3,4-diol | (2R,3S,5R)-2-[(6-amino-5-nitropyrimidin-4-yl)amino]-5-(hydroxymethyl)oxolane-3,4-diol | 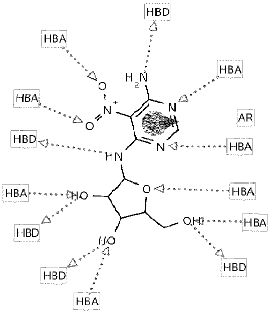 |
| 9 | 91572539 | (3R,4R,5R)-2-[(6-Amino-5-nitropyrimidin-4-yl)amino]-5-(hydroxymethyl)oxolane-3,4-diol | (3R,4R,5R)-2-[(6-amino-5-nitropyrimidin-4-yl)amino]-5-(hydroxymethyl)oxolane-3,4-diol | 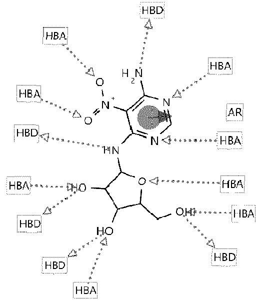 |
| 10 | 13770464 4 | (3S,4R,5S)-2-[(6-Amino-5-nitropyrimidin-4-yl)amino]-5-(hydroxymethyl)oxolane-3,4-diol | (3S,4R,5S)-2-[(6-amino-5-nitropyrimidin-4-yl)amino]-5-(hydroxymethyl)oxolane-3,4-diol | 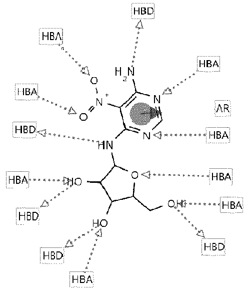 |

The inventors carried out a fingerprint Tanimoto-based 2-dimensional similarity search (Tanimoto threshold = 95%) of clitocine (compound CID129111) and identified 43 compounds (**compounds 1-43**, including clitocine as compound 1) as disclosed in **Table 2.**

**Table 2. List of identified compounds 1-43**

| **Compound** | **PubChem CID** | **Compound name** | **IUPAC name** |
|---|---|---|---|
| 1 | 129111 | Clitocine | (2R,3R,4S,5R)-2-[(6-amino-5-nitropyrimidin-4-yl)amino]-5-(hydroxymethyl)oxolane-3,4-diol |
| 11 | 9795869 | (2R,3S,4R,5R)-2-Aminomethyl-5-(6-amino-5-nitro-pyrimidin-4-ylamino)-tetrahydro-furan-3,4-diol | (2R,3S,4R,5R)-2-(aminomethyl)-5-[(6-amino-5-nitropyrimidin-4-yl)amino]oxolane-3,4-diol |
| 12 | 10017723 | (2R,3S,4R,5R)-5-[(6-Amino-5-nitropyrimidin-4-yl)amino]-2-(hydroxymethyl)-3-methyloxolane-3,4-diol | (2R,3S,4R,5R)-5-[(6-amino-5-nitropyrimidin-4-yl)amino]-2-(hydroxymethyl)-3-methyloxolane-3,4-diol |
| 13 | 10038815 | (2R,3S,5R)-5-[(6-Amino-5-nitropyrimidin-4-yl)amino]-2-(hydroxymethyl)oxolan-3-ol | (2R,3S,5R)-5-[(6-amino-5-nitropyrimidin-4-yl)amino]-2-(hydroxymethyl)oxolan-3-ol |
| 14 | 10065264 | (2R,3R,4S,5R)-2-[[6-(2-Hydroxyethylamino)-5-nitropyrimidin-4-yl]amino]-5-(hydroxymethyl)oxolane-3,4-diol | (2R,3R,4S,5R)-2-[[6-(2-hydroxyethylamino)-5-nitropyrimidin-4-yl]amino]-5-(hydroxymethyl)oxolane-3,4-diol |
| 15 | 10085552 | (2R,3S,4S,5R)-2-[(6-Amino-5-nitropyrimidin-4-yl)amino]-4-fluoro-5-(hydroxymethyl)oxolan-3-ol | (2R,3S,4S,5R)-2-[(6-amino-5-nitropyrimidin-4-yl)amino]-4-fluoro-5-(hydroxymethyl)oxolan-3-ol |
| 16 | 10334340 | (2R,3R,4S,5S)-2-[(6-Amino-5-nitropyrimidin-4-yl)amino]-5-(fluoromethyl)oxolane-3,4-diol | (2R,3R,4S,5S)-2-[(6-amino-5-nitropyrimidin-4-yl)amino]-5-(fluoromethyl)oxolane-3,4-diol |
| 17 | 10378472 | (2R,3R,4S,5R)-2-(6-Amino-5-nitro-pyrimidin-4-ylamino)-5-methyl-tetrahydro-furan-3,4-diol | (2R,3R,4S,5R)-2-[(6-amino-5-nitropyrimidin-4-yl)amino]-5-methyloxolane-3,4-diol |
| 18 | 10402587 | (2R,3R,4S,5R)-2-[(6-Amino-5-nitropyrimidin-4-yl)amino]-5-(2-hydroxyethyl)oxolane-3,4-diol | (2R,3R,4S,5R)-2-[(6-amino-5-nitropyrimidin-4-yl)amino]-5-(2-hydroxyethyl)oxolane-3,4-diol |
| 19 | 10424240 | (2R,3S,4R,5R)-2-[(6-Amino-5-nitropyrimidin-4-yl)amino]-4-fluoro-5-(hydroxymethyl)oxolan-3-ol | (2R,3S,4R,5R)-2-[(6-amino-5-nitropyrimidin-4-yl)amino]-4-fluoro-5-(hydroxymethyl)oxolan-3-ol |
| 20 | 10424919 | (2R,3R,4S,5R)-2-[(6-Amino-5-nitropyrimidin-4-yl)amino]-5-(1-hydroxyethyl)oxolane-3,4-diol | (2R,3R,4S,5R)-2-[(6-amino-5-nitropyrimidin-4-yl)amino]-5-(1-hydroxyethyl)oxolane-3,4-diol |
| 21 | 10446869 | (2R,3R,4S,5R)-5-[(6-Amino-5-nitropyrimidin-4-yl)amino]-4-fluoro-2-(hydroxymethyl)oxolan-3-ol | (2R,3R,4S,5R)-5-[(6-amino-5-nitropyrimidin-4-yl)amino]-4-fluoro-2-(hydroxymethyl)oxolan-3-ol |
| 22 | 10470946 | (2R,3R,4S,5R)-2-[[6-(Ethylamino)-5-nitropyrimidin-4-yl]amino]-5-(hydroxymethyl)oxolane-3,4-diol | (2R,3R,4S,5R)-2-[[6-(ethylamino)-5-nitropyrimidin-4-yl]amino]-5-(hydroxymethyl)oxolane-3,4-diol |
| 2 | 11644921 | 2-[(6-Amino-5-nitropyrimidin-4-yl)amino]-5-(hydroxymethyl)oxolane-3,4-diol | 2-[(6-amino-5-nitropyrimidin-4-yl)amino]-5-(hydroxymethyl)oxolane-3,4-diol |
| 3 | 13965719 | (3R,4S,5R)-2-[(6-Amino-5-nitropyrimidin-4-yl)amino]-5-(hydroxymethyl)oxolane-3,4-diol | (3R,4S,5R)-2-[(6-amino-5-nitropyrimidin-4-yl)amino]-5-(hydroxymethyl)oxolane-3,4-diol |
| 4 | 13965721 | (2R,3R,4R,5R)-2-[(6-Amino-5-nitropyrimidin-4-yl)amino]-5-(hydroxymethyl)oxolane-3,4-diol | (2R,3R,4R,5R)-2-[(6-amino-5-nitropyrimidin-4-yl)amino]-5-(hydroxymethyl)oxolane-3,4-diol |
| 23 | 58996355 | (2R,3R,4R,5R)-5-[(6-Amino-5-nitropyrimidin-4-yl)amino]-4-fluoro-2-(hydroxymethyl)oxolan-3-ol | (2R,3R,4R,5R)-5-[(6-amino-5-nitropyrimidin-4-yl)amino]-4-fluoro-2-(hydroxymethyl)oxolan-3-ol |
| 24 | 58996356 | (2R,3R,4S,5R)-2-[[6-(Dimethylamino)-5-nitropyrimidin-4-yl]amino]-5-(hydroxymethyl)oxolane-3,4-diol | (2R,3R,4S,5R)-2-[[6-(dimethylamino)-5-nitropyrimidin-4-yl]amino]-5-(hydroxymethyl)oxolane-3,4-diol |
| 25 | 58996357 | (2R,3S,4R,5R)-2-(Hydroxymethyl)-5-[[6-(methylamino)-5-nitropyrimidin-4-yl]amino]oxolane-3,4-diol | (2R,3S,4R,5R)-2-(hydroxymethyl)-5-[[6-(methylamino)-5-nitropyrimidin-4-yl]amino]oxolane-3,4-diol |
| 5 | 58996359 | (2S,3S,4R,5S)-2-[(6-Amino-5-nitropyrimidin-4-yl)amino]-5-(hydroxymethyl)oxolane-3,4-diol | (2S,3S,4R,5S)-2-[(6-amino-5-nitropyrimidin-4-yl)amino]-5-(hydroxymethyl)oxolane-3,4-diol |
| 26 | 58996362 | (2R,3S,5R)-2-[(6-Amino-5-nitropyrimidin-4-yl)amino]-4,4-difluoro-5-(hydroxymethyl)oxolan-3-ol | (2R,3S,5R)-2-[(6-amino-5-nitropyrimidin-4-yl)amino]-4,4-difluoro-5-(hydroxymethyl)oxolan-3-ol |
| 6 | 60139991 | (2S,3R,4S,5R)-2-[(6-Amino-5-nitropyrimidin-4-yl)amino]-5-(hydroxymethyl)oxolane-3,4-diol | (2S,3R,4S,5R)-2-[(6-amino-5-nitropyrimidin-4-yl)amino]-5-(hydroxymethyl)oxolane-3,4-diol |
| 27 | 69221289 | 5-[(6-Amino-5-nitropyrimidin-4-yl)amino]-4-fluoro-2-(hydroxymethyl)oxolan-3-ol | 5-[(6-amino-5-nitropyrimidin-4-yl)amino]-4-fluoro-2-(hydroxymethyl)oxolan-3-ol |
| 28 | 69225115 | 2-[(6-Amino-5-nitropyrimidin-4-yl)amino]-5-(2-hydroxyethyl)oxolane-3,4-diol | 2-[(6-amino-5-nitropyrimidin-4-yl)amino]-5-(2-hydroxyethyl)oxolane-3,4-diol |
| 29 | 69225288 | 2-[(6-Amino-5-nitropyrimidin-4-yl)amino]-5-methyloxolane-3,4-diol | 2-[(6-amino-5-nitropyrimidin-4-yl)amino]-5-methyloxolane-3,4-diol |
| 30 | 69225538 | 5-[(6-Amino-5-nitropyrimidin-4-yl)amino]-2-(hydroxymethyl)oxolan-3-ol | 5-[(6-amino-5-nitropyrimidin-4-yl)amino]-2-(hydroxymethyl)oxolan-3-ol |
| 31 | 69225577 | 2-[(6-Amino-5-nitropyrimidin-4-yl)amino]-4,4-difluoro-5-(hydroxymethyl)oxolan-3-ol | 2-[(6-amino-5-nitropyrimidin-4-yl)amino]-4,4-difluoro-5-(hydroxymethyl)oxolan-3-ol |
| 32 | 69225578 | 2-(Aminomethyl)-5-[(6-amino-5-nitropyrimidin-4-yl)amino]oxolane-3,4-diol | 2-(aminomethyl)-5-[(6-amino-5-nitropyrimidin-4-yl)amino]oxolane-3,4-diol |
| 33 | 69225751 | 2-[(6-Amino-5-nitropyrimidin-4-yl)amino]-5-(1-hydroxyethyl)oxolane-3,4-diol | 2-[(6-amino-5-nitropyrimidin-4-yl)amino]-5-(1-hydroxyethyl)oxolane-3,4-diol |
| 34 | 69226163 | 5-[(6-Amino-5-nitropyrimidin-4-yl)amino]-2-(hydroxymethyl)-3-methyloxolane-3,4-diol | 5-[(6-amino-5-nitropyrimidin-4-yl)amino]-2-(hydroxymethyl)-3-methyloxolane-3,4-diol |
| 35 | 69226282 | 2-[(6-Amino-5-nitropyrimidin-4-yl)amino]-4-fluoro-5-(hydroxymethyl)oxolan-3-ol | 2-[(6-amino-5-nitropyrimidin-4-yl)amino]-4-fluoro-5-(hydroxymethyl)oxolan-3-ol |
| 36 | 69717518 | (3R,4S,5R)-2-[[6-(Ethylamino)-5-nitropyrimidin-4-yl]amino]-5-(hydroxymethyl)oxolane-3,4-diol | (3R,4S,5R)-2-[[6-(ethylamino)-5-nitropyrimidin-4-yl]amino]-5-(hydroxymethyl)oxolane-3,4-diol |
| 37 | 69717520 | (2S,3R,4S,5R)-2-[[6-(Ethylamino)-5-nitropyrimidin-4-yl]amino]-5-(hydroxymethyl)oxolane-3,4-diol | (2S,3R,4S,5R)-2-[[6-(ethylamino)-5-nitropyrimidin-4-yl]amino]-5-(hydroxymethyl)oxolane-3,4-diol |
| 7 | 71136912 | (2R,5R)-2-[(6-Amino-5-nitropyrimidin-4-yl)amino]-5-(hydroxymethyl)oxolane-3,4-diol | (2R,5R)-2-[(6-amino-5-nitropyrimidin-4-yl)amino]-5-(hydroxymethyl)oxolane-3,4-diol |
| 8 | 90294771 | (2R,3S,5R)-2-[(6-Amino-5-nitropyrimidin-4-yl)amino]-5-(hydroxymethyl)oxolane-3,4-diol | (2R,3S,5R)-2-[(6-amino-5-nitropyrimidin-4-yl)amino]-5-(hydroxymethyl)oxolane-3,4-diol |
| 38 | 90860724 | (2R,3R,5S)-2-[(6-Amino-5-nitropyrimidin-4-yl)amino]-5-[azido(hydroxy)methyl]oxolan-3-ol | (2R,3R,5S)-2-[(6-amino-5-nitropyrimidin-4-yl)amino]-5-[azido(hydroxy)methyl]oxolan-3-ol |
| 39 | 90900219 | (2R,3S,4R)-2-(Hydroxymethyl)-5-[[6-(methylamino)-5-nitropyrimidin-4-yl]amino]oxolane-3,4-diol | (2R,3S,4R)-2-(hydroxymethyl)-5-[[6-(methylamino)-5-nitropyrimidin-4-yl]amino]oxolane-3,4-diol |
| 40 | 91074498 | (3R,4S,5R)-2-[[6-(Dimethylamino)-5-nitropyrimidin-4-yl]amino]-5-(hydroxymethyl)oxolane-3,4-diol | (3R,4S,5R)-2-[[6-(dimethylamino)-5-nitropyrimidin-4-yl]amino]-5-(hydroxymethyl)oxolane-3,4-diol |
| 41 | 91088753 | (3R,4S,5R)-2-[[6-(2-Hydroxyethylamino)-5-nitropyrimidin-4-yl]amino]-5-(hydroxymethyl)oxolane-3,4-diol | (3R,4S,5R)-2-[[6-(2-hydroxyethylamino)-5-nitropyrimidin-4-yl]amino]-5-(hydroxymethyl)oxolane-3,4-diol |
| 42 | 91521750 | (3R,4S,5S)-2-[(6-Amino-5-nitropyrimidin-4-yl)amino]-5-(fluoromethyl)oxolane-3,4-diol | (3R,4S,5S)-2-[(6-amino-5-nitropyrimidin-4-yl)amino]-5-(fluoromethyl)oxolane-3,4-diol |
| 9 | 91572539 | (3R,4R,5R)-2-[(6-Amino-5-nitropyrimidin-4-yl)amino]-5-(hydroxymethyl)oxolane-3,4-diol | (3R,4R,5R)-2-[(6-amino-5-nitropyrimidin-4-yl)amino]-5-(hydroxymethyl)oxolane-3,4-diol |
| 43 | 10123433 6 | (2R,3R,4S,5R)-2-[[6-[Ethyl(methyl)amino]-5-nitropyrimidin-4-yl]amino]-5-(hydroxymethyl)oxolane-3,4-diol | (2R,3R,4S,5R)-2-[[6-[ethyl(methyl)amino]-5-nitropyrimidin-4-yl]amino]-5-(hydroxymethyl)oxolane-3,4-diol |
| 10 | 13770464 4 | (3S,4R,5S)-2-[(6-Amino-5-nitropyrimidin-4-yl)amino]-5-(hydroxymethyl)oxolane-3,4-diol | (3S,4R,5S)-2-[(6-amino-5-nitropyrimidin-4-yl)amino]-5-(hydroxymethyl)oxolane-3,4-diol |

The inventors carried out a fingerprint Tanimoto-based 2-dimensional similarity search (Tanimoto threshold = 90%) of clitocine (compound CID129111) and identified 76 compounds (**compounds 1-76**, including clitocine as compound 1) as disclosed in **Table 3.**

**Table 3. List of identified compounds 1-76**

| **Compound** | **PubChem CID** | **Compound name** | **IUPAC name** |
|---|---|---|---|
| 1 | 129111 | Clitocine | (2R,3R,4S,5R)-2-[(6-amino-5-nitropyrimidin-4-yl)amino]-5-(hydroxymethyl)oxolane-3,4-diol |
| 44 | 592060 | 9-beta-d-Arabinofuranosyl-adenine-N'-oxide | 2-(6-amino-9-oxidopurin-9-ium-9-yl)-5-(hydroxymethyl)oxolane-3,4-diol |
| 11 | 9795869 | (2R,3S,4R,5R)-2-Aminomethyl-5-(6-amino-5-nitro-pyrimidin-4-ylamino)-tetrahydrofuran-3,4-diol | (2R,3S,4R,5R)-2-(aminomethyl)-5-[(6-amino-5-nitropyrimidin-4-yl)amino]oxolane-3,4-diol |
| 45 | 9971159 | (2R,4S,5R)-5-[(6-Amino-5-nitropyrimidin-4-yl)amino]-4-hydroxy-2-(hydroxymethyl)oxolan-3-one | (2R,4S,5R)-5-[(6-amino-5-nitropyrimidin-4-yl)amino]-4-hydroxy-2-(hydroxymethyl)oxolan-3-one |
| 12 | 10017723 | (2R,3S,4R,5R)-5-[(6-Amino-5-nitropyrimidin-4-yl)amino]-2-(hydroxymethyl)-3-methyloxolane-3,4-diol | (2R,3S,4R,5R)-5-[(6-amino-5-nitropyrimidin-4-yl)amino]-2-(hydroxymethyl)-3-methyloxolane-3,4-diol |
| 13 | 10038815 | (2R,3S,5R)-5-[(6-Amino-5-nitropyrimidin-4-yl)amino]-2-(hydroxymethyl)oxolan-3-ol | (2R,3S,5R)-5-[(6-amino-5-nitropyrimidin-4-yl)amino]-2-(hydroxymethyl)oxolan-3-ol |
| 14 | 10065264 | (2R,3R,4S,5R)-2-[[6-(2-Hydroxyethylamino)-5-nitropyrimidin-4-yl]amino]-5-(hydroxymethyl)oxolane-3,4-diol | (2R,3R,4S,5R)-2-[[6-(2-hydroxyethylamino)-5-nitropyrimidin-4-yl]amino]-5-(hydroxymethyl)oxolane-3,4-diol |
| 15 | 10085552 | (2R,3S,4S,5R)-2-[(6-Amino-5-nitropyrimidin-4-yl)amino]-4-fluoro-5-(hydroxymethyl)oxolan-3-ol | (2R,3S,4S,5R)-2-[(6-amino-5-nitropyrimidin-4-yl)amino]-4-fluoro-5-(hydroxymethyl)oxolan-3-ol |
| 46 | 10086834 | (2R,3R,4S,5R)-2-[(6-Amino-5-nitropyrimidin-4-yl)amino]-5-(azidomethyl)oxolane-3,4-diol | (2R,3R,4S,5R)-2-[(6-amino-5-nitropyrimidin-4-yl)amino]-5-(azidomethyl)oxolane-3,4-diol |
| 47 | 10107264 | (2R,3S,4R,5R)-2-(Hydroxymethyl)-5-[(5-nitropyrimidin-4-yl)amino]oxolane-3,4-diol | (2R,3S,4R,5R)-2-(hydroxymethyl)-5-[(5-nitropyrimidin-4-yl)amino]oxolane-3,4-diol |
| 16 | 10334340 | (2R,3R,4S,5S)-2-[(6-Amino-5-nitropyrimidin-4-yl)amino]-5-(fluoromethyl)oxolane-3,4-diol | (2R,3R,4S,5S)-2-[(6-amino-5-nitropyrimidin-4-yl)amino]-5-(fluoromethyl)oxolane-3,4-diol |
| 17 | 10378472 | (2R,3R,4S,5R)-2-(6-Amino-5-nitropyrimidin-4-ylamino)-5-methyl-tetrahydrofuran-3,4-diol | (2R,3R,4S,5R)-2-[(6-amino-5-nitropyrimidin-4-yl)amino]-5-methyloxolane-3,4-diol |
| 48 | 10381774 | [(3Ar,4R,6R,6aR)-4-[(6-amino-5-nitropyrimidin-4-yl)amino]-2,2-dimethyl-3a,4,6,6a-tetrahydrofuro[3,4-d][1,3]dioxol-6-yl]methanol | [(3aR,4R,6R,6aR)-4-[(6-amino-5-nitropyrimidin-4-yl)amino]-2,2-dimethyl-3a,4,6,6a-tetrahydrofuro[3,4-d][1,3]dioxol-6-yl]methanol |
| 18 | 10402587 | (2R,3R,4S,5R)-2-[(6-Amino-5-nitropyrimidin-4-yl)amino]-5-(2-hydroxyethyl)oxolane-3,4-diol | (2R,3R,4S,5R)-2-[(6-amino-5-nitropyrimidin-4-yl)amino]-5-(2-hydroxyethyl)oxolane-3,4-diol |
| 49 | 10402931 | (2R,3R,5R)-5-[(6-Amino-5-nitropyrimidin-4-yl)amino]-4,4-difluoro-2-(hydroxymethyl)oxolan-3-ol | (2R,3R,5R)-5-[(6-amino-5-nitropyrimidin-4-yl)amino]-4,4-difluoro-2-(hydroxymethyl)oxolan-3-ol |
| 19 | 10424240 | (2R,3S,4R,5R)-2-[(6-Amino-5-nitropyrimidin-4-yl)amino]-4-fluoro-5-(hydroxymethyl)oxolan-3-ol | (2R,3S,4R,5R)-2-[(6-amino-5-nitropyrimidin-4-yl)amino]-4-fluoro-5-(hydroxymethyl)oxolan-3-ol |
| 20 | 10424919 | (2R,3R,4S,5R)-2-[(6-Amino-5-nitropyrimidin-4-yl)amino]-5-(1-hydroxyethyl)oxolane-3,4-diol | (2R,3R,4S,5R)-2-[(6-amino-5-nitropyrimidin-4-yl)amino]-5-(1-hydroxyethyl)oxolane-3,4-diol |
| 50 | 10445180 | (2R,3R,4S,5R)-2-[(5,6-Diaminopyrimidin-4-yl)amino]-5-(hydroxymethyl)oxolane-3,4-diol | (2R,3R,4S,5R)-2-[(5,6-diaminopyrimidin-4-yl)amino]-5-(hydroxymethyl)oxolane-3,4-diol |
| 21 | 10446869 | (2R,3R,4S,5R)-5-[(6-Amino-5-nitropyrimidin-4-yl)amino]-4-fluoro-2-(hydroxymethyl)oxolan-3-ol | (2R,3R,4S,5R)-5-[(6-amino-5-nitropyrimidin-4-yl)amino]-4-fluoro-2-(hydroxymethyl)oxolan-3-ol |
| 22 | 10470946 | (2R,3R,4S,5R)-2-[[6-(Ethylamino)-5-nitropyrimidin-4-yl]amino]-5-(hydroxymethyl)oxolane-3,4-diol | (2R,3R,4S,5R)-2-[[6-(ethylamino)-5-nitropyrimidin-4-yl]amino]-5-(hydroxymethyl)oxolane-3,4-diol |
| 2 | 11644921 | 2-[(6-Amino-5-nitropyrimidin-4-yl)amino]-5-(hydroxymethyl)oxolane-3,4-diol | 2-[(6-amino-5-nitropyrimidin-4-yl)amino]-5-(hydroxymethyl)oxolane-3,4-diol |
| 51 | 11724091 | [(2R,3S,4R,5R)-5-[(6-Amino-5-nitropyrimidin-4-yl)amino]-3,4-dihydroxyoxolan-2-yl]methylacetate | [(2R,3S,4R,5R)-5-[(6-amino-5-nitropyrimidin-4-yl)amino]-3,4-dihydroxyoxolan-2-yl]methylacetate |
| 3 | 13965719 | (3R,4S,5R)-2-[(6-Amino-5-nitropyrimidin-4-yl)amino]-5-(hydroxymethyl)oxolane-3,4-diol | (3R,4S,5R)-2-[(6-amino-5-nitropyrimidin-4-yl)amino]-5-(hydroxymethyl)oxolane-3,4-diol |
| 4 | 13965721 | (2R,3R,4R,5R)-2-[(6-Amino-5-nitropyrimidin-4-yl)amino]-5-(hydroxymethyl)oxolane-3,4-diol | (2R,3R,4R,5R)-2-[(6-amino-5-nitropyrimidin-4-yl)amino]-5-(hydroxymethyl)oxolane-3,4-diol |
| 52 | 13965723 | [(3Ar,6R,6aR)-4-[(6-amino-5-nitropyrimidin-4-yl)amino]-2,2-dimethyl-3a,4,6,6a-tetrahydrofuro[3,4-d][1,3]dioxol-6-yl]methanol | [(3aR,6R,6aR)-4-[(6-amino-5-nitropyrimidin-4-yl)amino]-2,2-dimethyl-3a,4,6,6a-tetrahydrofuro[3,4-d][1,3]dioxol-6-yl]methanol |
| 53 | 15539937 | [(2R,3S)-5-[(6-Amino-5-nitropyrimidin-4-yl)amino]-3-[tert-butyl(dimethyl)silyl]oxyoxolan-2-yl]methanol | [(2R,3S)-5-[(6-amino-5-nitropyrimidin-4-yl)amino]-3-[tert-butyl(dimethyl)silyl]oxyoxolan-2-yl]methanol |
| 54 | 44317554 | (2R,3R,4S,5S)-2-[(6-Amino-5-nitropyrimidin-4-yl)amino]-5-(bromomethyl)oxolane-3,4-diol | (2R,3R,4S,5S)-2-[(6-amino-5-nitropyrimidin-4-yl)amino]-5-(bromomethyl)oxolane-3,4-diol |
| 55 | 54314153 | (2R,3R,5S)-2-(6-Amino-9-oxidopurin-9-ium-9-yl)-5-(hydroxymethyl)oxolan-3-ol | (2R,3R,5S)-2-(6-amino-9-oxidopurin-9-ium-9-yl)-5-(hydroxymethyl)oxolan-3-ol |
| 56 | 54341839 | (2R,3R,4S,5R)-2-(6-Amino-9-oxidopurin-9-ium-9-yl)-5-(hydroxymethyl)oxolane-3,4-diol | (2R,3R,4S,5R)-2-(6-amino-9-oxidopurin-9-ium-9-yl)-5-(hydroxymethyl)oxolane-3,4-diol |
| 23 | 58996355 | (2R,3R,4R,5R)-5-[(6-Amino-5-nitropyrimidin-4-yl)amino]-4-fluoro-2-(hydroxymethyl)oxolan-3-ol | (2R,3R,4R,5R)-5-[(6-amino-5-nitropyrimidin-4-yl)amino]-4-fluoro-2-(hydroxymethyl)oxolan-3-ol |
| 24 | 58996356 | (2R,3R,4S,5R)-2-[[6-(Dimethylamino)-5-nitropyrimidin-4-yl]amino]-5-(hydroxymethyl)oxolane-3,4-diol | (2R,3R,4S,5R)-2-[[6-(dimethylamino)-5-nitropyrimidin-4-yl]amino]-5-(hydroxymethyl)oxolane-3,4-diol |
| 25 | 58996357 | (2R,3S,4R,5R)-2-(Hydroxymethyl)-5-[[6-(methylamino)-5-nitropyrimidin-4-yl]amino]oxolane-3,4-diol | (2R,3S,4R,5R)-2-(hydroxymethyl)-5-[[6-(methylamino)-5-nitropyrimidin-4-yl]amino]oxolane-3,4-diol |
| 5 | 58996359 | (2S,3S,4R,5S)-2-[(6-Amino-5-nitropyrimidin-4-yl)amino]-5-(hydroxymethyl)oxolane-3,4-diol | (2S,3S,4R,5S)-2-[(6-amino-5-nitropyrimidin-4-yl)amino]-5-(hydroxymethyl)oxolane-3,4-diol |
| 26 | 58996362 | (2R,3S,5R)-2-[(6-Amino-5-nitropyrimidin-4-yl)amino]-4,4-difluoro-5-(hydroxymethyl)oxolan-3-ol | (2R,3S,5R)-2-[(6-amino-5-nitropyrimidin-4-yl)amino]-4,4-difluoro-5-(hydroxymethyl)oxolan-3-ol |
| 6 | 60139991 | (2S,3R,4S,5R)-2-[(6-Amino-5-nitropyrimidin-4-yl)amino]-5-(hydroxymethyl)oxolane-3,4-diol | (2S,3R,4S,5R)-2-[(6-amino-5-nitropyrimidin-4-yl)amino]-5-(hydroxymethyl)oxolane-3,4-diol |
| 27 | 69221289 | 5-[(6-Amino-5-nitropyrimidin-4-yl)amino]-4-fluoro-2-(hydroxymethyl)oxolan-3-ol | 5-[(6-amino-5-nitropyrimidin-4-yl)amino]-4-fluoro-2-(hydroxymethyl)oxolan-3-ol |
| 57 | 69222181 | 5-[(6-Amino-5-nitropyrimidin-4-yl)amino]-4,4-difluoro-2-(hydroxymethyl)oxolan-3-ol | 5-[(6-amino-5-nitropyrimidin-4-yl)amino]-4,4-difluoro-2-(hydroxymethyl)oxolan-3-ol |
| 58 | 69222394 | 2-(Hydroxymethyl)-5-[(5-nitropyrimidin-4-yl)amino]oxolane-3,4-diol | 2-(hydroxymethyl)-5-[(5-nitropyrimidin-4-yl)amino]oxolane-3,4-diol |
| 59 | 69223701 | Aceticacid;[(2R,3S,4R,5R)-5-[(6-amino-5-nitropyrimidin-4-yl)amino]-3,4-dihydroxyoxolan-2-yl]methyl acetate | aceticacid;[(2R,3S,4R,5R)-5-[(6-amino-5-nitropyrimidin-4-yl)amino]-3,4-dihydroxyoxolan-2-yl]methyl acetate |
| 28 | 69225115 | 2-[(6-Amino-5-nitropyrimidin-4-yl)amino]-5-(2-hydroxyethyl)oxolane-3,4-diol | 2-[(6-amino-5-nitropyrimidin-4-yl)amino]-5-(2-hydroxyethyl)oxolane-3,4-diol |
| 60 | 69225277 | 5-[(6-Amino-5-nitropyrimidin-4-yl)amino]-4-hydroxy-2-(hydroxymethyl)oxolan-3-one | 5-[(6-amino-5-nitropyrimidin-4-yl)amino]-4-hydroxy-2-(hydroxymethyl)oxolan-3-one |
| 29 | 69225288 | 2-[(6-Amino-5-nitropyrimidin-4-yl)amino]-5-methyloxolane-3,4-diol | 2-[(6-amino-5-nitropyrimidin-4-yl)amino]-5-methyloxolane-3,4-diol |
| 30 | 69225538 | 5-[(6-Amino-5-nitropyrimidin-4-yl)amino]-2-(hydroxymethyl)oxolan-3-ol | 5-[(6-amino-5-nitropyrimidin-4-yl)amino]-2-(hydroxymethyl)oxolan-3-01 |
| 31 | 69225577 | 2-[(6-Amino-5-nitropyrimidin-4-yl)amino]-4,4-difluoro-5-(hydroxymethyl)oxolan-3-ol | 2-[(6-amino-5-nitropyrimidin-4-yl)amino]-4,4-difluoro-5-(hydroxymethyl)oxolan-3-ol |
| 32 | 69225578 | 2-(Aminomethyl)-5-[(6-amino-5-nitropyrimidin-4-yl)amino]oxolane-3,4-diol | 2-(aminomethyl)-5-[(6-amino-5-nitropyrimidin-4-yl)amino]oxolane-3,4-diol |
| 33 | 69225751 | 2-[(6-Amino-5-nitropyrimidin-4-yl)amino]-5-(1-hydroxyethyl)oxolane-3,4-diol | 2-[(6-amino-5-nitropyrimidin-4-yl)amino]-5-(1-hydroxyethyl)oxolane-3,4-diol |
| 34 | 69226163 | 5-[(6-Amino-5-nitropyrimidin-4-yl)amino]-2-(hydroxymethyl)-3-methyloxolane-3,4-diol | 5-[(6-amino-5-nitropyrimidin-4-yl)amino]-2-(hydroxymethyl)-3-methyloxolane-3,4-diol |
| 35 | 69226282 | 2-[(6-Amino-5-nitropyrimidin-4-yl)amino]-4-fluoro-5-(hydroxymethyl)oxolan-3-ol | 2-[(6-amino-5-nitropyrimidin-4-yl)amino]-4-fluoro-5-(hydroxymethyl)oxolan-3-ol |
| 61 | 69234609 | (2R,3S,4R,5R)-2-(Hydroxymethyl)-5-[(5-nitropyrimidin-2-yl)amino]oxolane-3,4-diol | (2R,3S,4R,5R)-2-(hydroxymethyl)-5-[(5-nitropyrimidin-2-yl)amino]oxolane-3,4-diol |
| 36 | 69717518 | (3R,4S,5R)-2-[[6-(Ethylamino)-5-nitropyrimidin-4-yl]amino]-5-(hydroxymethyl)oxolane-3,4-diol | (3R,4S,5R)-2-[[6-(ethylamino)-5-nitropyrimidin-4-yl]amino]-5-(hydroxymethyl)oxolane-3,4-diol |
| 37 | 69717520 | (2S,3R,4S,5R)-2-[[6-(Ethylamino)-5-nitropyrimidin-4-yl]amino]-5-(hydroxymethyl)oxolane-3,4-diol | (2S,3R,4S,5R)-2-[[6-(ethylamino)-5-nitropyrimidin-4-yl]amino]-5-(hydroxymethyl)oxolane-3.4-diol |
| 62 | 69722004 | Aceticacid;[(2R,3S,4R)-5-[(6-amino-5-nitropyrimidin-4-yl)amino]-3,4-dihydroxyoxolan-2-yl]methyl acetate | aceticacid;[(2R,3S,4R)-5-[(6-amino-5-nitropyrimidin-4-yl)amino]-3,4-dihydroxyoxolan-2-yl]methyl acetate |
| 63 | 69722005 | [(2R,3S,4R)-5-[(6-Amino-5-nitropyrimidin-4-yl)amino]-3,4-dihydroxyoxolan-2-yl]methyl acetate | [(2R,3S,4R)-5-[(6-amino-5-nitropyrimidin-4-yl)amino]-3,4-dihydroxyoxolan-2-yl]methylacetate |
| 64 | 69773457 | 2-[5-[(6-Amino-5-nitropyrimidin-4-yl)amino]oxolan-2-yl]ethanol | 2-[5-[(6-amino-5-nitropyrimidin-4-yl)amino]oxolan-2-yl]ethanol |
| 65 | 70917149 | 5-[(5,6-Diaminopyrimidin-4-yl)amino]-2-(hydroxymethyl)oxolan-3-ol | 5-[(5,6-diaminopyrimidin-4-yl)amino]-2-(hydroxymethyl)oxolan-3-ol |
| 66 | 71051136 | (3R,4S,5R)-2-[(5,6-Diaminopyrimidin-4-yl)amino]-5-(hydroxymethyl)oxolane-3,4-diol | (3R,4S,5R)-2-[(5,6-diaminopyrimidin-4-yl)amino]-5-(hydroxymethyl)oxolane-3,4-diol |
| 7 | 71136912 | (2R,5R)-2-[(6-Amino-5-nitropyrimidin-4-yl)amino]-5-(hydroxymethyl)oxolane-3,4-diol | (2R,5R)-2-[(6-amino-5-nitropyrimidin-4-yl)amino]-5-(hydroxymethyl)oxolane-3,4-diol |
| 67 | 71163925 | [(2R,3S,4R)-5-[(6-Amino-5-nitropyrimidin-4-yl)amino]-3,4-dihydroxyoxolan-2-yl]methyl dimethylphosphate | [(2R,3S,4R)-5-[(6-amino-5-nitropyrimidin-4-yl)amino]-3,4-dihydroxyoxolan-2-yl]methyl dimethylphosphate |
| 68 | 71221536 | (2R,3S,5R)-5-[(5,6-Diaminopyrimidin-4-yl)amino]-4-(2-hydroxyethoxy)-2-(hydroxymethyl)oxolan-3-ol | (2R,3S,5R)-5-[(5,6-diaminopyrimidin-4-yl)amino]-4-(2-hydroxyethoxy)-2-(hydroxymethyl)oxolan-3-ol |
| 69 | 77996097 | 2-[(5,6-Diaminopyrimidin-4-yl)amino]-5-(hydroxymethyl)oxolane-3,4-diol | 2-[(5,6-diaminopyrimidin-4-yl)amino]-5-(hydroxymethyl)oxolane-3,4-diol |
| 70 | 88887092 | [(2S,5R)-5-[[6-Amino-5-(hydroxyamino)pyrimidin-4-yl]amino]oxolan-2-yl]methanol | [(2S,5R)-5-[[6-amino-5-(hydroxyamino)pyrimidin-4-yl]amino]oxolan-2-yl]methanol |
| 71 | 89226248 | 2-[(2S)-5-[(6-Amino-5-nitropyrimidin-4-yl)amino]oxolan-2-yl]ethanol | 2-[(2S)-5-[(6-amino-5-nitropyrimidin-4-yl)amino]oxolan-2-yl]ethanol |
| 8 | 90294771 | (2R,3S,5R)-2-[(6-Amino-5-nitropyrimidin-4-yl)amino]-5-(hydroxymethyl)oxolane-3,4-diol | (2R,3S,5R)-2-[(6-amino-5-nitropyrimidin-4-yl)amino]-5-(hydroxymethyl)oxolane-3,4-diol |
| 72 | 90695347 | (3R,4S,5R)-2-[(6-Amino-5-nitropyrimidin-4-yl)amino]-5-(azidomethyl)oxolane-3,4-diol | (3R,4S,5R)-2-[(6-amino-5-nitropyrimidin-4-yl)amino]-5-(azidomethyl)oxolane-3,4-diol |
| 38 | 90860724 | (2R,3R,5S)-2-[(6-Amino-5-nitropyrimidin-4-yl)amino]-5-[azido(hydroxy)methyl]oxolan-3-ol | (2R,3R,5S)-2-[(6-amino-5-nitropyrimidin-4-yl)amino]-5-[azido(hydroxy)methyl]oxolan-3-ol |
| 39 | 90900219 | (2R,3S,4R)-2-(Hydroxymethyl)-5-[[6-(methylamino)-5-nitropyrimidin-4-yl]amino]oxolane-3,4-diol | (2R,3S,4R)-2-(hydroxymethyl)-5-[[6-(methylamino)-5-nitropyrimidin-4-yl]amino]oxolane-3,4-diol |
| 40 | 91074498 | (3R,4S,5R)-2-[[6-(Dimethylamino)-5-nitropyrimidin-4-yl]amino]-5-(hydroxymethyl)oxolane-3,4-diol | (3R,4S,5R)-2-[[6-(dimethylamino)-5-nitropyrimidin-4-yl]amino]-5-(hydroxymethyl)oxolane-3,4-diol |
| 41 | 91088753 | (3R,4S,5R)-2-[[6-(2-Hydroxyethylamino)-5-nitropyrimidin-4-yl]amino]-5-(hydroxymethyl)oxolane-3,4-diol | (3R,4S,5R)-2-[[6-(2-hydroxyethylamino)-5-nitropyrimidin-4-yl]amino]-5-(hydroxymethyl)oxolane-3,4-diol |
| 42 | 91521750 | (3R,4S,5S)-2-[(6-Amino-5-nitropyrimidin-4-yl)amino]-5-(fluoromethyl)oxolane-3,4-diol | (3R,4S,5S)-2-[(6-amino-5-nitropyrimidin-4-yl)amino]-5-(fluoromethyl)oxolane-3,4-diol |
| 9 | 91572539 | (3R,4R,5R)-2-[(6-Amino-5-nitropyrimidin-4-yl)amino]-5-(hydroxymethyl)oxolane-3,4-diol | (3R,4R,5R)-2-[(6-amino-5-nitropyrimidin-4-yl)amino]-5-(hydroxymethyl)oxolane-3,4-diol |
| 43 | 101234336 | (2R,3R,4S,5R)-2-[[6-[Ethyl(methyl)amino]-5-nitropyrimidin-4-yl]amino]-5-(hydroxymethyl)oxolane-3,4-diol | (2R,3R,4S,5R)-2-[[6-[ethyl(methyl)amino]-5-nitropyrimidin-4-yl]amino]-5-(hydroxymethyl)oxolane-3,4-diol |
| 73 | 101262263 | N-[4-Amino-6-[[(2R,3R,4S,5R)-3,4-dihydroxy-5-(hydroxymethyl)oxolan-2-yl]amino]pyrimidin-5-yl]formamide | N-[4-amino-6-[[(2R,3R,4S,5R)-3,4-dihydroxy-5-(hydroxymethyl)oxolan-2-yl]amino]pyrimidin-5-yl]formamide |
| 74 | 135019247 | [(2S,3R,5R)-5-[(6-Amino-5-nitropyrimidin-4-yl)amino]-3-azidooxolan-2-yl]methanol | [(2S,3R,5R)-5-[(6-amino-5-nitropyrimidin-4-yl)amino]-3-azidooxolan-2-yl]methanol |
| 75 | 135258154 | 2-[[6-Amino-5-(methylamino)pyrimidin-4-yl]amino]-5-(hydroxymethyl)oxolan-3-ol | 2-[[6-amino-5-(methylamino)pyrimidin-4-yl]amino]-5-(hydroxymethyl)oxolan-3-ol |
| 10 | 137704644 | (3S,4R,5S)-2-[(6-Amino-5-nitropyrimidin-4-yl)amino]-5-(hydroxymethyl)oxolane-3,4-diol | (3S,4R,5S)-2-[(6-amino-5-nitropyrimidin-4-yl)amino]-5-(hydroxymethyl)oxolane-3,4-diol |
| 76 | 141378328 | (2R,3S,4R,5R)-2-(Hydroxymethyl)-5-[6-(methylamino)-9-oxidopurin-9-ium-9-yl]oxolane-3,4-diol | (2R,3S,4R,5R)-2-(hydroxymethyl)-5-[6-(methylamino)-9-oxidopurin-9-ium-9-yl]oxolane-3,4-diol |

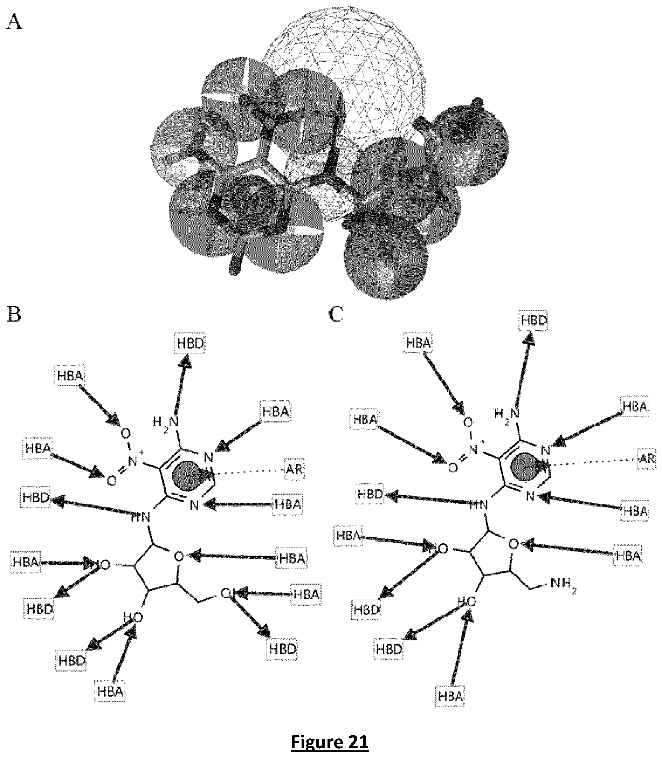

The inventors carried out a fingerprint Tanimoto-based 3-dimensional similarity search (Tanimoto threshold = 100%) of clitocine (compound CID129111) and identified 267 compounds. Then the inventors predicted the pharmacophore features of 15 compounds including clitocine that were randomly selected from said 267 identified compounds, as disclosed in Table 4, and analyzed the pharmacophore alignment using feature and alignment point. For example, the inventors showed a similarity of pharmacophore features between clitocine (CID_129111 corresponding to compound 1 of Table 1) and CID9795869 [(2R,3S,4R,5R)-2-Aminomethyl-5-(6-amino-5-nitropyrimidin-4-ylamino)-tetrahydro-furan-3,4-diol corresponding to compound 11 of Table 2] (**Figure 21**).

**Table 4. List of the 15 selected compounds, i.e. compounds 1, 11 and 77-90.**

| **Compound** | **PubChem CID** | **Compound name** | **IUPAC name** | **Pharmacophore features** |
|---|---|---|---|---|
| 77 | 6245 | Tubercidin | (2R,3R,4S,5R)-2-(4-aminopyrrolo[2,3-d]pyrimidin-7-yl)-5-(hydroxymethyl)oxolane -3,4-diol | 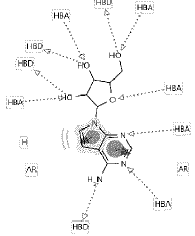 |
| 78 | 8975 | 2-Fluoroadenosine | (2R,3R,4S,5R)-2-(6-amino-2-fluoropurin-9-yl)-5-(hydroxymethyl)oxolane -3,4-diol | 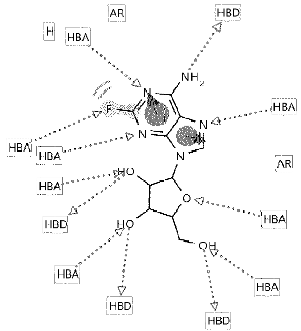 |
| 79 | 60961 | Adenosine | (2R,3R,4S,5R)-2-(6-aminopurin-9-yl)-5-(hydroxymethyl)oxolane -3,4-diol | 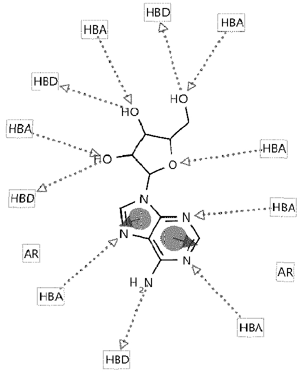 |
| 80 | 439182 | 5'-Deoxyadenosine | (2R,3R,4S,5R)-2-(6-aminopurin-9-yl)-5-methyloxolane-3,4-diol | 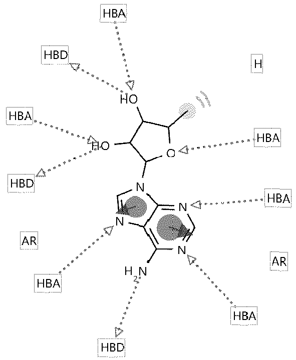 |
| 81 | 447199 | Formycin | (2S,3R,4S,5R)-2-(7-amino-2H-pyrazolo[4,3-d]pyrimidin-3-yl)-5-(hydroxymethyl)oxolane -3,4-diol | 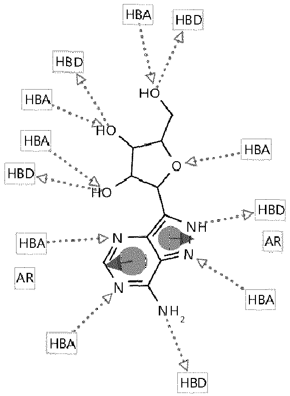 |
| 82 | 448403 | 5'-Fluoro-5'-deoxyadenosine | (2R,3R,4S,5S)-2-(6-aminopurin-9-yl)-5-(fluoromethyl)oxolane-3,4-diol | 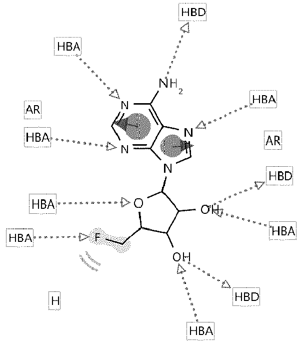 |
| 83 | 472638 | 3-Cyclopentene-1,2-diol, 5-(6-amino-9H-purin-9-yl)-3-((1R)-1-hydroxy-2-propynyl)-, (1S,2R,5R)- | (1S,2R,5R)-5-(6-aminopurin-9-yl)-3-[(1R)-1-hydroxyprop-2-ynyl]cyclopent-3-ene-1,2-diol | 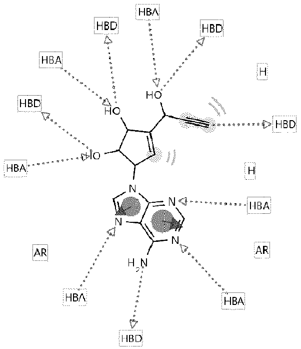 |
| 11 | 9795869 | (2R,3S,4R,5R)-2-Aminomethyl-5-(6-amino-5-nitropyrimidin-4-ylamino)-tetrahydro-furan-3,4-diol | (2R,3S,4R,5R)-2-(aminomethyl)-5-[(6-amino-5-nitropyrimidin-4-yl)amino]oxolane-3,4-diol | 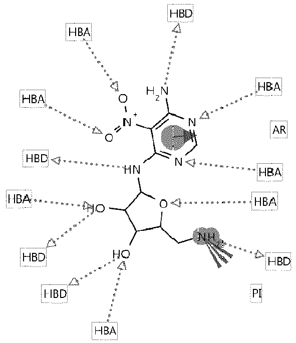 |
| 84 | 10334232 | (2R,3R,4S,5R)-2-[(6-Amino-5-nitropyridazin-4-yl)amino]-5-(hydroxymethyl)oxola ne-3,4-diol | (2R,3R,4S,5R)-2-[(6-amino-5-nitropyridazin-4-yl)amino]-5-(hydroxymethyl)oxolane -3,4-diol | 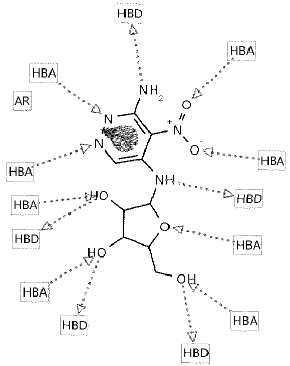 |
| 85 | 10446747 | (2R,3R,4S,5R)-2-[(5-Amino-4-nitropyridazin-3-yl)amino]-5-(hydroxymethyl)oxolane-3,4-diol | (2R,3R,4S,5R)-2-[(5-amino-4-nitropyridazin-3-yl)amino]-5-(hydroxymethyl)oxolane -3,4-diol | 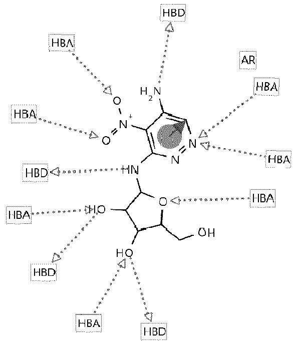 |
| 86 | 10753174 | 5'-Deoxytubercidin | (2R,3R,4S,5R)-2-(4-aminopyrrolo[2,3-d]pyrimidin-7-yl)-5-methyloxolane-3,4-diol | 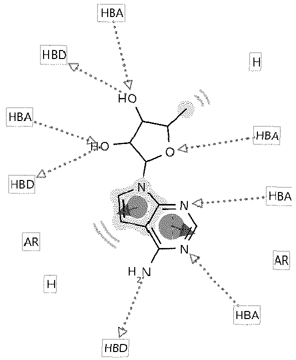 |
| 87 | 46876860 | (3R,4S,5S)-2-(6-Aminopurin-9-yl)-5-(d ifluoromethyl)oxolane-3,4-diol | (3R,4S,5S)-2-(6-aminopurin-9-yl)-5-(d ifluoromethyl)oxolane-3,4-diol | 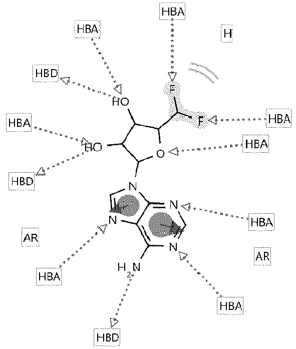 |
| 88 | 101875585 | (2R,3S,4S,5S)-2-(6-Aminopurin-9-yl)-4,5-difluoro-5-(hydroxymethyl)oxolan-3-ol | (2R,3S,4S,5S)-2-(6-aminopurin-9-yl)-4,5-difluoro-5-(hydroxymethyl)oxolan-3-ol | 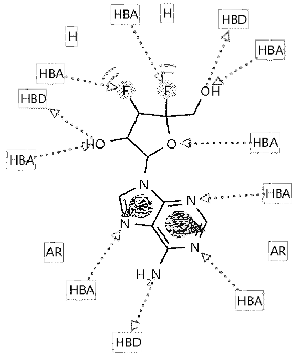 |
| 89 | 102006363 | 5,5-Difluoromethyl Adenosine | (2R,4S,5S)-2-(6-aminopurin-9-yl)-5-(difluoromethyl)oxolane-3,4-diol | 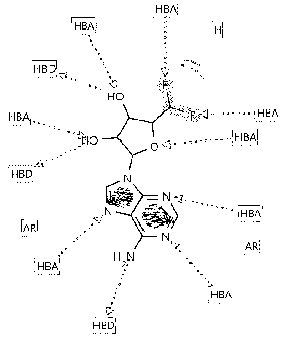 |
| 90 | 118717403 | (2R,3S,4R,5S)-2-(6-Aminopurin-9-yl)-4,5-difluoro-5-(hydroxymethyl)oxolan-3-ol | (2R,3S,4R,5S)-2-(6-aminopurin-9-yl)-4,5-difluoro-5-(hydroxymethyl)oxolan-3-ol | 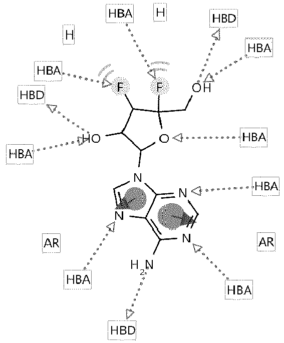 |

Other features and advantages of the invention will be apparent from the examples which follow and will also be illustrated in the figures.

### BRIEF DESCRIPTION OF THE FIGURES

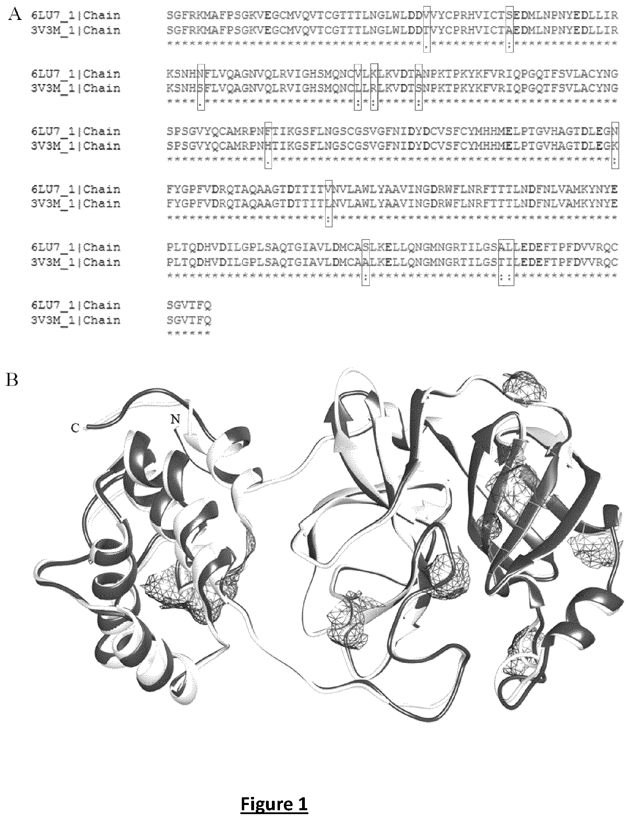

**Figure 1****. Sequence and structural differences between SARS-CoV-2 and SARS-CoV-1. 1A.** The multiple sequence alignment of SARS-CoV-2 (6LU7) and SARS-CoV-1 (3V3M) showed 96% of sequence identity as (*). Other dissimilar residues are indicated in the box. **1B.** Structural superimposition of the crystal structures of SARS-CoV-2 (White) and SARS-CoV-1 (Black). The modified sequence is shown in surface mesh view between the two proteins.

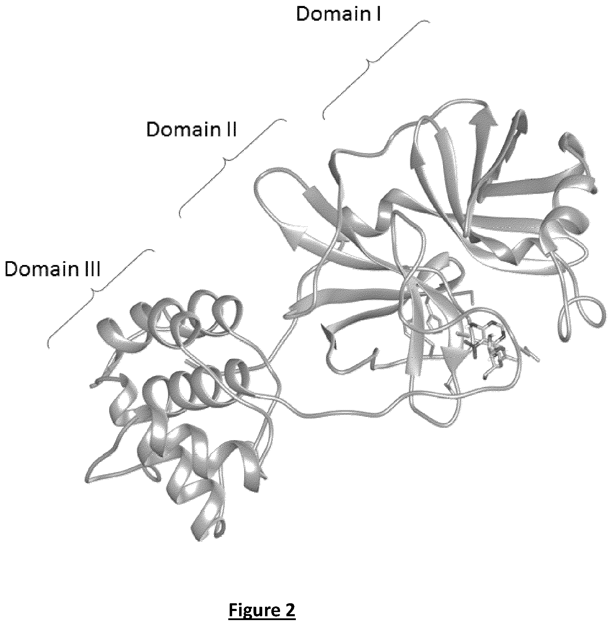

**Figure 2****. Protein domains of SARS-CoV-2 with CID_129111.** The ligand interacted towards the substrate-binding pocket of SARS-CoV-2.

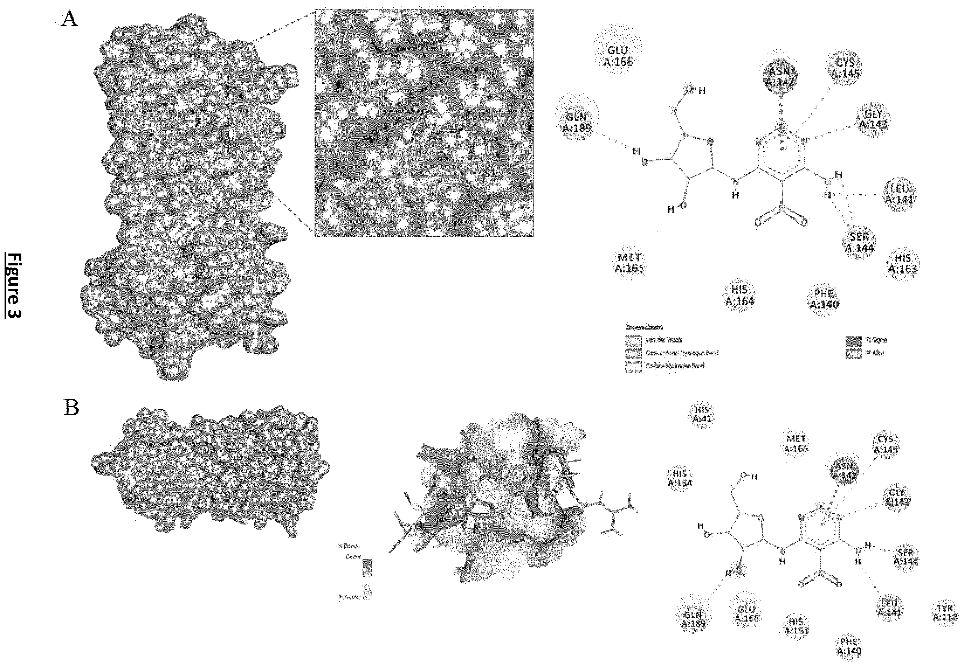

**Figure 3****. Molecular docking of 6LU7 and 7BQY with CID_129111. 3A.** Molecular docking of 6LU7 showed the best-fit interactions with the S1 position. Interactions: Van der Waals: F140, H163, H164; Conventional Hydrogen Bond: L141, G143, S144, Q189; Carbon Hydrogen Bond: E166, M165; Pi-Sigma: N142; Pi-Alkyl: C145. **3B.** Molecular docking of 7BQY showed a similar interaction with few other sites (S1, S2, S4). Interactions: Van der Waals: H41, Y118, F140, H163, H164, E166; Conventional Hydrogen Bond: L141, G143, S144, Q189; Carbon Hydrogen Bond: M165; Pi-Sigma: N142; Pi-Alkyl: C145. Both docking analyses 3A and 3B showed the Pi-sigma (N142) and Pi-Alkyl (C145) interactions.

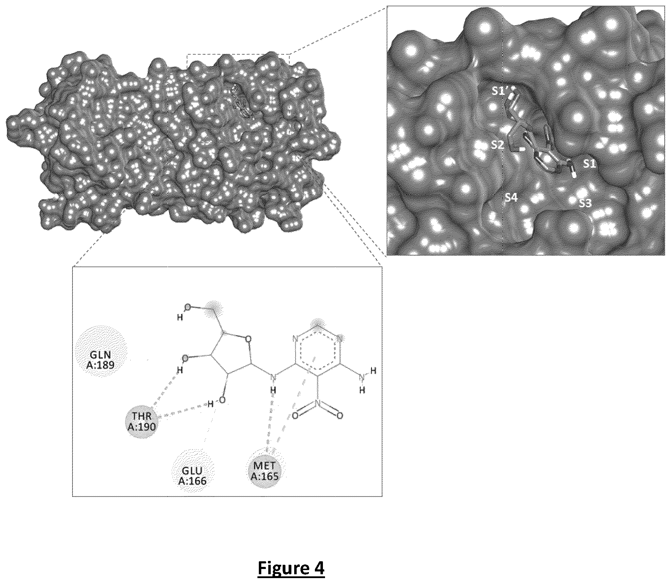

**Figure 4****. Repetition of molecular docking of 6LU7 with CID_129111.** Said repetition showed the conventional H-bond (M165, T190) and carbon H-bond (E166, Q189) interactions as well as the Pi-Alkyl (M165) interaction in the S1', S1, S2, S4 positions corresponding to the substrate-binding pocket in SARS-CoV-2.

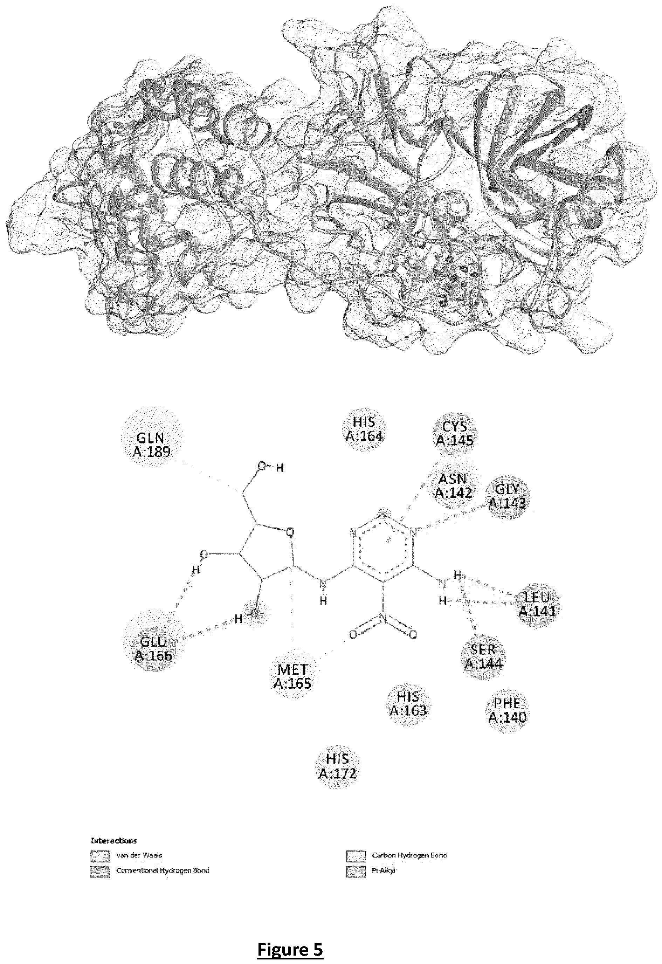

**Figure 5****. Interaction of 6LU7A with CID_129111 (Site-specific docking).** Several conventional H bond (L141, G143, S144, E166) and carbon H-bond (M165, Q189) interactions as well as a Pi-Alkyl (C145) interaction were observed in the catalytic site. Van der Waals interactions were also observed (F140, N142, H163, H164, H172).

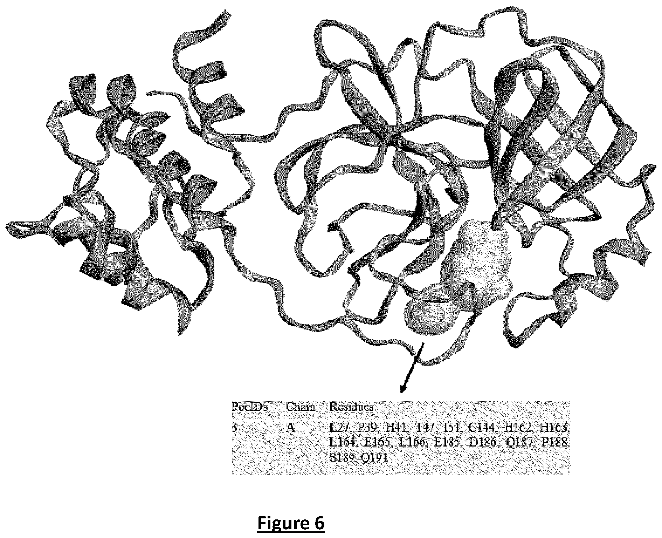

**Figure 6****. Binding site analysis of FIPV (5EU8).** The substrate-binding site (white cartoon bubble shape) was predicted by CASTp server, and the binding site residues were indicated in the box.

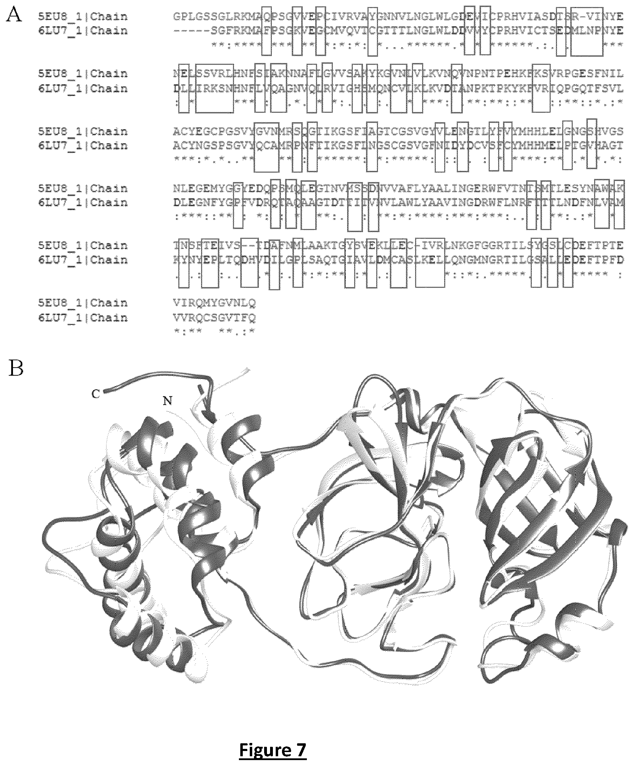

**Figure 7****. Sequence and structural differences between SARS-CoV-2 and FIPV. 7A.** The multiple sequence alignment of SARS-CoV-2 (6LU7) and FIPV (5EU8) showed 44.70% of sequence identity as (*). Other dissimilar residues are indicated in the box. **7B.** Structural superimposition of the crystal structures of SARS-CoV-2 (White) and FIPV (Black).

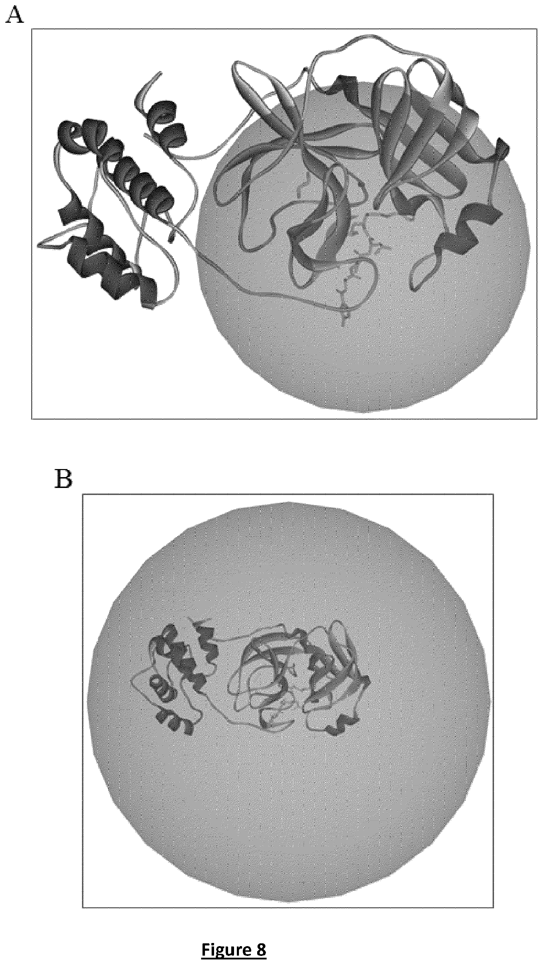

**Figure 8****. Two different approaches of FIPV (5EU8) docking analysis. 8A.** Site-specific docking of SBD pocket with 25Å radius. **8B.** Global flexible ligand binding with the large radius of 60Å.

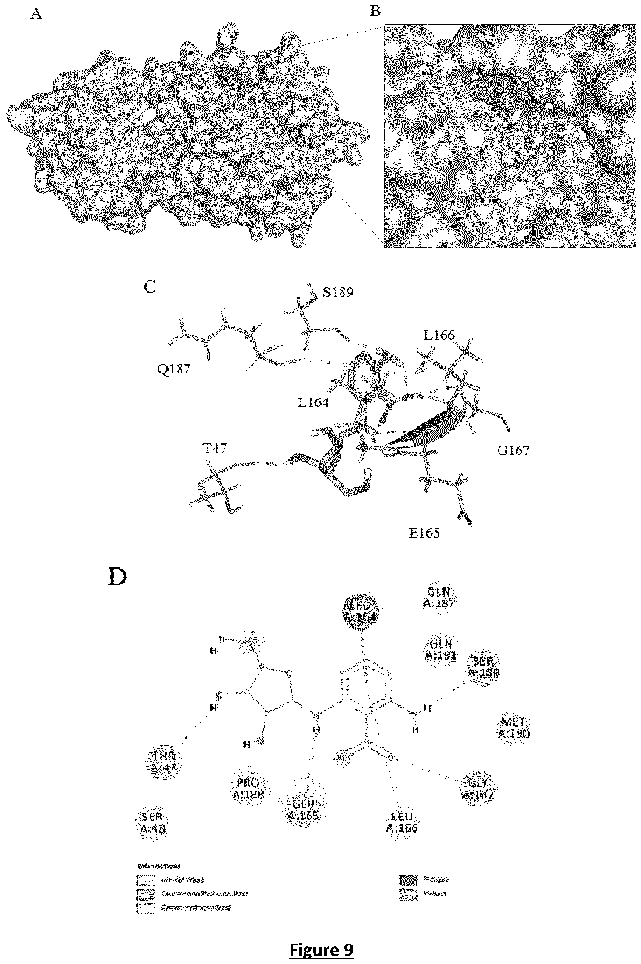

**Figure 9****. Site-specific ligand interaction with FIPV (5EU8). 9A-9B** showed the protein-ligand interaction in molecular surface. **9C.** SBD site interaction of main H-bond residues with Pi-Sigma (L164), Pi-Alkyl (L166) residues on surface. **9D.** 2Dmap of CID_129111 residue interaction with FIPV. Interactions: Van der Waals: S48, P188, M190, Q191; Conventional Hydrogen Bond: T47, E165, G167, S189; Carbon Hydrogen Bond: L166, Q187; Pi-Sigma: L164; Pi-Alkyl: L166.

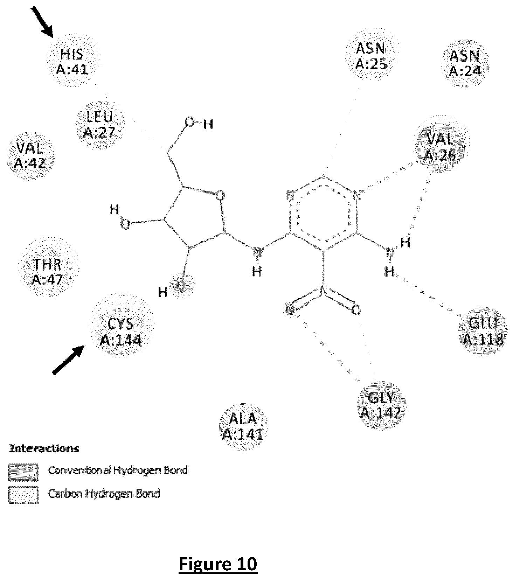

**Figure 10****. 2Dmap of CID_129111 interaction with FIPV (5EU8).** The conventional and carbon H-bond interactions were indicated in dotted lines. Conventional Hydrogen Bond: V26, E118, G142; Carbon Hydrogen Bond: N25, H41, G142. The ligand was present in the catalytic site, as shown by the marked residues H41 and C144 (see the arrows).

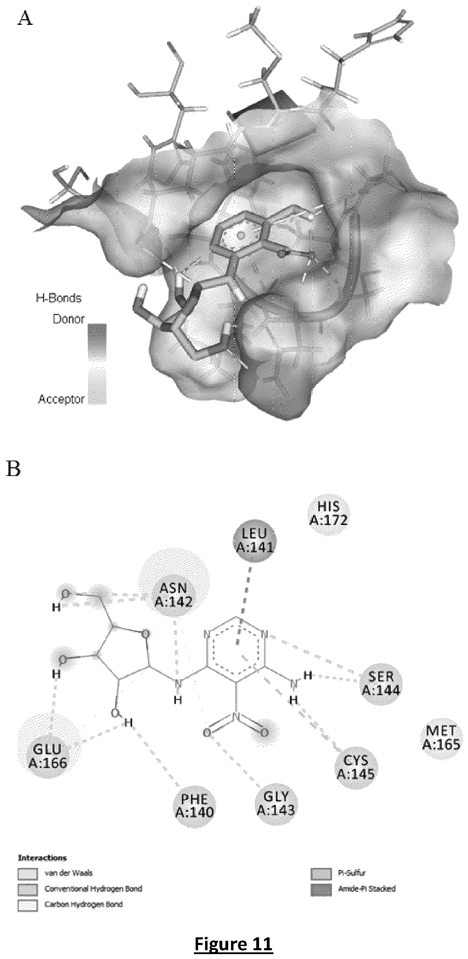

**Figure 11****. Site-specific docking of SARS-CoV-1 (3V3M) with CID_129111. 11A.** Surface view of residual interaction. **11B.** 2Dmap of CID_129111 interaction. The Pi-Sulfur (C145) and the Amide-Pi-stacked (L141) contacts are shown in dark. Several conventional and carbon H-bond interactions were observed. Interactions: Van der Waals: M165, H172; Conventional Hydrogen Bond: F140, G143, S144, C145, N142, E166; Carbon Hydrogen Bond: N142, E166; Pi-Sulfur: C145; Amide-Pi Stacked: L141.

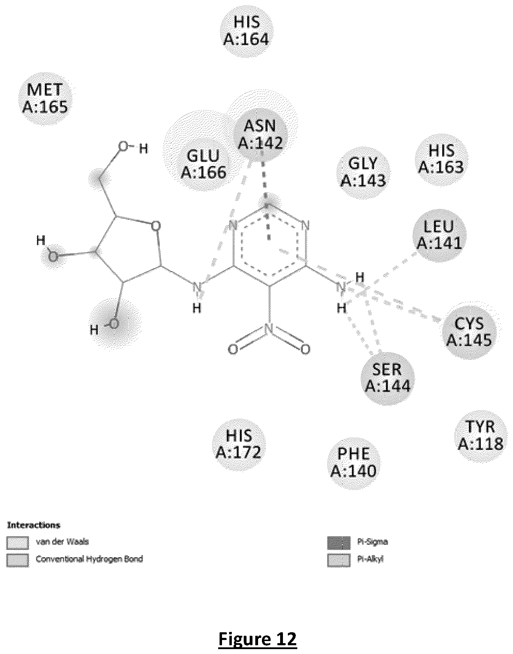

**Figure 12****. 2D map of CID_129111 interaction with SARS-CoV-1 (3V3M).** N142 and C145 had the H-bond and Pi interactions. Other H-bond interactions are L141 and S144. Interactions: Van der Waals: Y118, F140, G143, H163, M165, H164, E166, H172; Conventional Hydrogen Bond: L141, N142, S144, C145; Pi-Sigma: N142; Pi-Alkyl: C145.

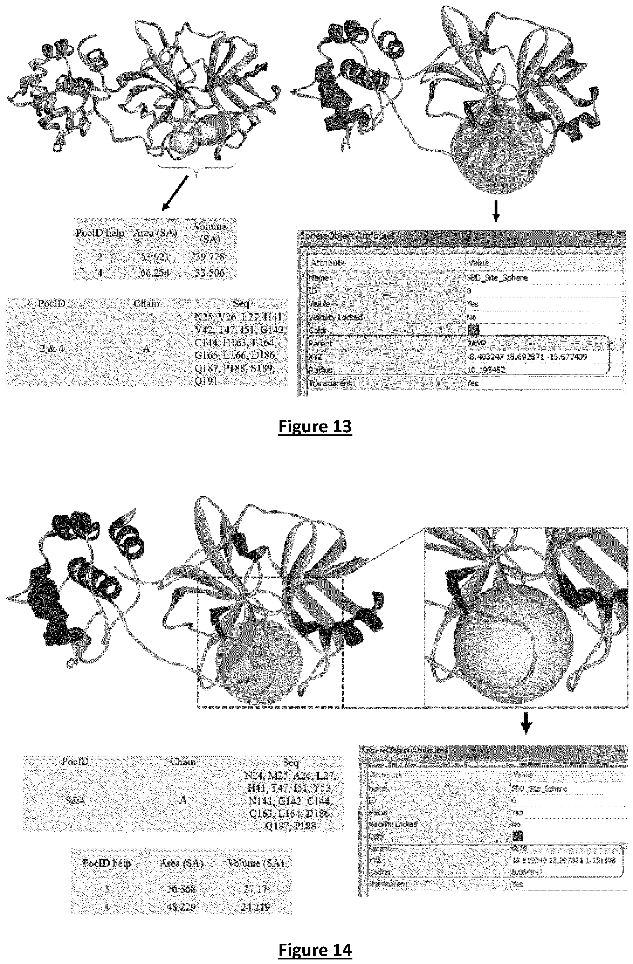

**Figure 13****. Binding site properties and the docking grid map of TGEV protein (2AMP).** The binding sites of TGEV PocID2 (White) and PocID4 (Black) are indicated.
**Figure 14****. Binding site properties and the docking grid map of PEDV protein (6L70).**

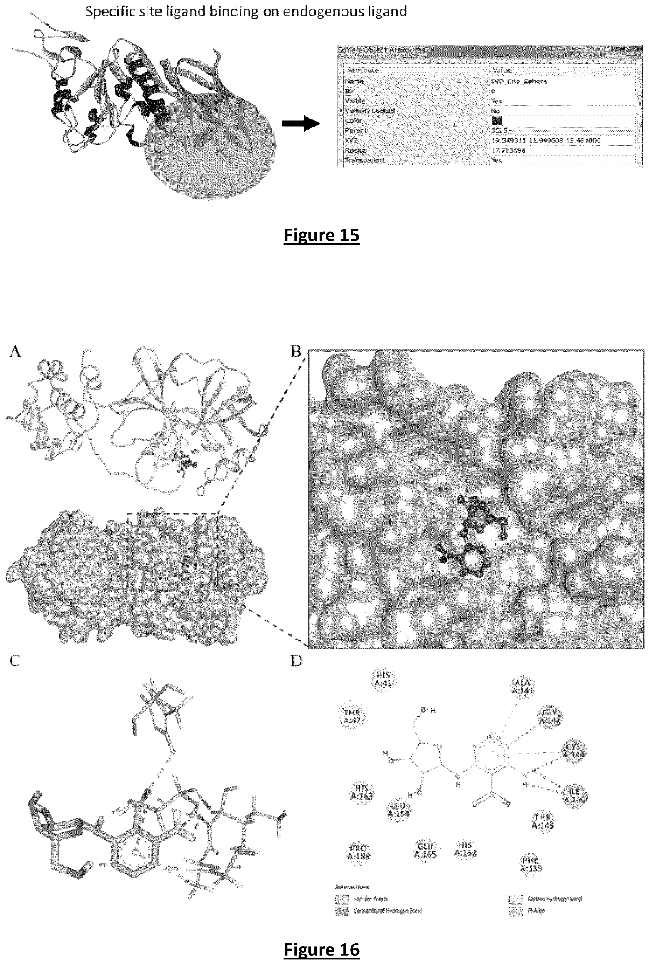

**Figure 15****. Binding site properties and the docking grid map of BCoV-HE protein (3CL5).**
**Figure 16****. Site-specific ligand interaction with TGEV (2AMP). 16A-16B** showed the protein-ligand interaction in molecular surface. **16C.** SBD site interaction of main H-bond residues with Pi -Alkyl residues on surface. **16D.** 2Dmap of CID_129111 residue interaction with TGEV. Interactions: Van der Waals: H41, T47, F139, T143, H163, L164, E165 P188; Conventional Hydrogen Bond: 1140, G142 C144; Carbon Hydrogen Bond: H162; Pi-Alkyl: A141.

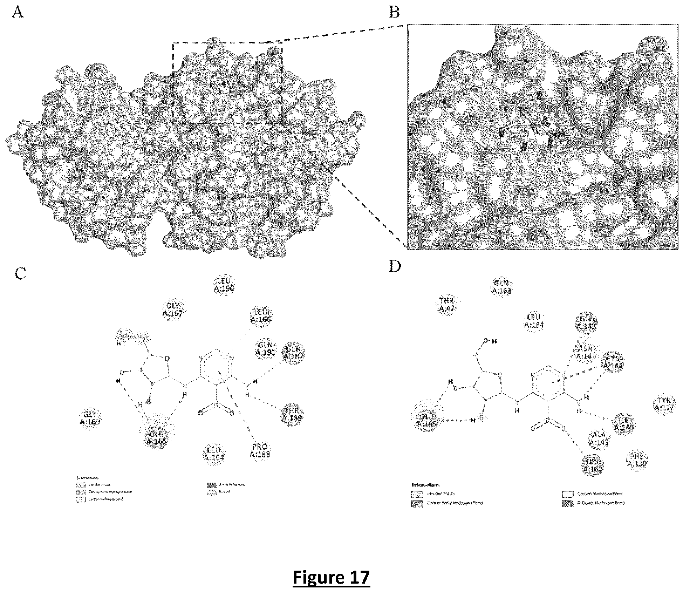

**Figure 17****. Site-specific ligand interaction with PEDV (6L70). 17A-17B** showed the protein-ligand interaction in molecular surface. **17C.** SBD site interaction of docking pose 1 with H-bond residues with Amide-Pi-stacked residue. Interactions: Van der Waals: L164, G167, G169, L190, Q191; Conventional Hydrogen Bond: E165, Q187, T189; Carbon Hydrogen Bond: P188; Pi-Alkyl: L166; Amide-Pi Stacked: P188. **17D.** SBD site interaction of docking pose 2 with H-bond residues with Pi-Alkyl residue. Interactions: Van der Waals: T47, Y117, F139, N141, A143, Q163; Conventional Hydrogen Bond: 1140, G142, C144, H162, E165; Carbon Hydrogen Bond: L164; Pi-Donor H Bond: C144.

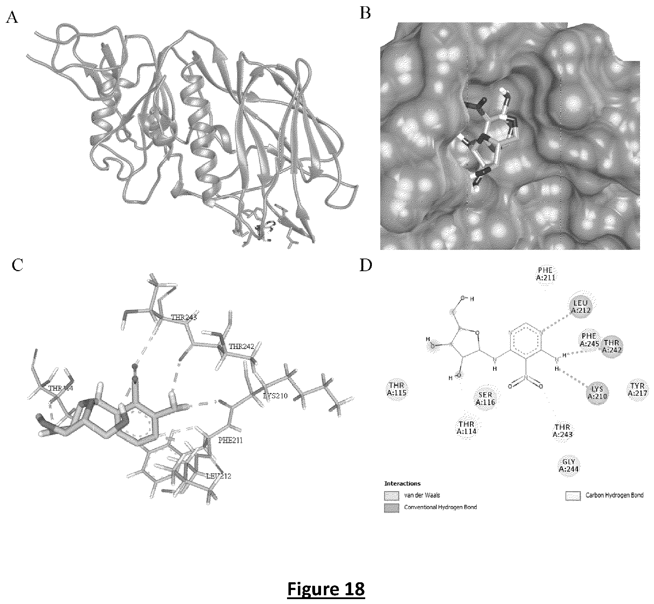

**Figure 18****. Site-specific ligand interaction with BCoV-HE (3CL5). 18A-18B** showed the protein-ligand interaction in molecular surface. **18C.** Molecular view of ligand interaction with H-bond residues. **18D.** The 2D map of ligand interaction. Interactions: Van der Waals: T115, S116, Y217, G244, F245; Conventional Hydrogen Bond: K210, L212, T242; Carbon Hydrogen Bond: T114, F211, T243.
**Figure 19****. 3D Structure of clitocine (CID_129111).**
**Figure 20****. Pharmacophore structure of clitocine (CID_129111).**
**Figure 21****. Similarity of pharmacophore features between clitocine (CID_129111) and CID9795869 ((2R,3S,4R,5R)-2-Aminomethyl-5-(6-amino-5-nitro-pyrimidin-4-ylamino)-tetrahydro-furan-3,4-diol; compound 11 of Table 2). 21A.** Pharmacophre alignment of both compounds. **21B.** Pharmacophore features of CID_129111. **21C.** Pharmacophore features of CID_9795869. (HBA = Hydrogen Bond Acceptor; HBD = Hydrogen Bond Donor; AR = Aromatic Ring).

### EXAMPLES

### Example 1. Study of molecular interactions of high-resolution experimental structure of SARS-CoV-2, i.e. COVID-19 virus, Mpro with CID-129111 using docking analysis.

### Computational Methods

### Protein and Ligand collection

The crystallography structure of SARS-CoV-2 Mpro in complex with an inhibitor N3 at two different resolutions was obtained from the protein databank [PDB ID: 6LU7 (2.16 A°) & 7BQY (1.7 A°)] (Jin et al., Nature, 2020, 1-5*).* The 3D structure of clitocine (CID_129111) was collected from PubChem database.

The SARS-CoV-2 Mpro [PDB ID: 6LU7 (2.16 A°)] has an amino acid sequence as defined in SEQ ID NO: 1.

The SARS-CoV-2 Mpro [PDB ID: 7BQY (1.7 A°)] has an amino acid sequence as defined in SEQ ID NO: 4.

### Molecular docking study

The molecular docking calculations were carried out using DockingServer (Bikadi and Hazai, J. Cheminform. 2009, 1, 15*)*. Gasteiger partial charges were added to the ligand atoms. Non-polar hydrogen atoms were merged, and rotatable bonds were defined. Docking calculations were carried out on 6LU7 and 7BQY protein models. Essential hydrogen atoms, Kollman united atom type charges, and solvation parameters were added with the aid of AutoDock tools (Morris, G. M., et al. J. Comput. Chem., 1998, 19(14), 1639-1662). AutoDock parameter set- and distance-dependent dielectric functions were used in the calculation of the van der Waals and the electrostatic terms, respectively. Docking simulations were performed using the Lamarckian genetic algorithm (LGA) and the Solis & Wets local search method (Solis, F. J., & Wets, R. J. B. Math. Oper. Res., 1981, 6(1), 19-30)*.* Initial position, orientation, and torsions of the ligand molecules were set randomly.

### Docking Analysis I and II (SARS-CoV-2_6LU7A with CID_129111)

The affinity (grid) maps of 60 x 60 x 60 Å (nx, ny, and nz) grid points and cx = -26.1, cy=12.68, cz = 58.85 were selected as a grid box. The 0.375 Å spacing was generated using the Autogrid program (Morris, G. M., et al. J. Comput. Chem., 1998, 19(14), 1639-1662) was optimized using MMFF94 gasteiger charge calculation method and the ligand had MMFF94 energy of 28.19533 kcal/mol. All rotatable torsions were released during docking. Each docking experiment was derived from 100 different runs that were set to terminate after a maximum of 2500000 energy evaluations. The population size was set to 150. During the search, a translational step of 0.2 Å, and quaternion and torsion steps of 5 were applied. The docking process was repeated for the validation of the protein-ligand interactions with the same parameters. Both analyses (I and II) were performed as a flexible docking approach with the large radius range (60 Å).

### Docking Analysis III (SARS-CoV-2_7BQYA with CID_129111)

The grid maps of 30 x 30 x 30 Å (nx, ny, nz) grid points and cx = 9.103367, cy = -0.928102, cz = 22.691245 were selected as a grid box. The 0.375 Å spacing was generated using the Autogrid program (Morris, G. M., et al. J. Comput. Chem., 1998, 19(14), 1639-1662). The docking simulation was performed for the flexible ligands into the site-specific binding pocket (radius 30 Å). Furthermore, the same parameters for ligand and docking run were used for the analysis. The residual interactions were analyzed using UCSF chimera, LigPlot and Discovery studio visualizer tool.

### Docking analysis IV (Site-specific-low radius)

The grid maps of 20 x 20 x 20 Å (nx, ny, nz) grid points and cx = - 10.711837, cy = 12.411388, cz = 68.831286 were selected as a grid box. The 0.375 Å spacing was generated using the Autogrid program (Morris, G. M., et al. J. Comput. Chem., 1998, 19(14), 1639-1662). The docking simulation was performed for the flexible ligands into the site-specific binding pocket (low radius 20 Å).

### Results and Discussion

### Docking analysis

A sequence alignment as well as the structural superimposition of the crystal structures of SARS-CoV-2 and SARS-CoV-1 are shown in **Figure 1****.**

The Mpro of SARS-CoV-2 (PDB: 6LU7A and 7BQYA) was docked to CID_129111 using Autodock tools. The inventors analyzed out the compound CID_129111 with the recently deposited crystal structures having 2 different resolutions (PDB: 6LU7A and 7BQYA). The inventors selected the best ligand interaction file based on hydrogen bond, docking pose and the ligand binding free energy values, and obtained top hit ligand-binding poses from the different docking analyses.

Previously, the substrate-binding pocket of SARS-CoV-2 was reported as P1, P1', P2, P3, P4 and P5 sites of N3 inhibitor and the report suggested that the compound cinaserin occupied the substrate-binding pocket by interacting with H41 and E166 residues (Jin et al., Nature, 2020, 1-5)*.* Another report suggested that the compound ZINC000541677852 was maintaining the key interactions of Q189, and M149 as hydrophobic and the C145, H164, E166 as H-bond interactions (Ton et al., Molecular Informatics, 2020, 1-8).

The present results also revealed that the ligand had the interaction in the same binding site of N3 inhibitor (Domain II) (**Figure 2**). The first docking (Rank 1) had a lowest estimation binding free energy (-6.36 kcal/mol (6LU7A) and -6.92 kcal/mol (7BQYA)) with subsite 1 (S1) corresponding to N3 inhibitor substrate-binding pocket (Jin et al., Nature, 2020, 1-5). Furthermore, the residual interactions of ligand showed several H-bonds (L141, G143, S144, M165, Q189), Pi-Alkyl (C145) and Pi-sigma (N142) interactions (**Figure 3****; Table 5 below**). A similar interaction was reported in earlier studies (Ton et al., Molecular Informatics, 2020, 1-8, 2020; Jin et al., Nature, 2020, 1-5). In general, the Pi-sigma bonds were exhibiting stronger interactions than the Pi bonds, and the Pi-Alkyl bonds were depicting the least overlapping in the orbitals.

Then the inventors repeated the same analysis with 200 runs of docking simulation using SARS-CoV-2 structure (6LU7A), the ligand interaction showed the similar estimation binding-free energy of -6.33 kcal/mol in the N3 substrate binding pocket with outstanding H-bond (E166, M165, Q189, T190) residual interactions (**Figure 4**).

Once again, the inventors performed the molecular docking analysis of 6LU7 to reconfirm the residual interaction within the lowest radius grid size or the site-specific interaction of ligand towards the protein SARS-CoV-2. The results reconfirmed that the ligand served strong double H-bond interaction with S1 position (L141 and E166) and one H-bond with S144. Furthermore, the other carbon H-bond interaction was displayed in the S2 (M165) and S4 (G189) positions (**Figure 5**). All the docking results are shown in **Table 5.**

**Table 5. The residual interaction and the binding-free energy value of SARS-CoV-2 Mpro-ligand docking.**

| **Rank** | **PDB_ID _Chain** | **Est. Free Energy of Binding** | **Est. Inhibition Constant, Ki** | **H bond** | **Hydrophobic interactions (Pi-Alkyl/Pi-Sigma)** | **Grid radius (A)** | **Frequency** | **Int. Surface** |
|---|---|---|---|---|---|---|---|---|
| 1 | 6LU7_A | -6.36 kcal/mol | 21.88 uM | L141, G143, S144, M165, Q189 | N142, C145 | 60 x 60 x 60 | 1% | 561.784 |
| Repeat | 6LU7_A | -6.33 kcal/mol | 22.85 uM | E166, M165, Q189, T190 | M165 | 60 x 60 x 60 | 1% | 560.077 |
| 1 | 7BQY_ A | -6.92 kcal/mol | 8.53 uM | L141, G143, S144, M165, Q189 | N142, C145 | 30 x 30 x 30 | 3% | 606.11 |
| 1 | 6LU7_A | -6.89 kcal/mol | 8.97 uM | L141, G143, S144, M165, E166, Q189 | C145 | 20 x 20 x 20 | 11% | 599.449 |

Earlier computational studies suggested many compounds (Lopinavir, Ritonavir, Beclabuvir, Saquinavir, Nelfinavir, Atazanavir, Ledipasvir, Elbasvir, Efavirnez) as potential drug targets for SARS-CoV-2 using computational docking simulation with preliminary clinical data (Wang et al., Clinical characteristics and therapeutic procedure for four cases with 2019 novel coronavirus pneumonia receiving combined Chinese and Western medicine treatment. Biosci Trends. 2020*;* Sekhar Talluri. (2020) Virtual Screening Based Prediction of Potential Drugs for COVID-19. Preprint. Doi: 10.20944/preprints202002.0418.v2*;* Xu et al., 2020, Nelfinavir was predicted to be a potential inhibitor of 2019-nCov main protease by an integrative approach combining homology modelling, molecular docking and binding free energy calculation. bioRxiv. doi:10.1101/2020.01.27.921627*;* Beck et al., 2020, Predicting commercially available antiviral drugs that may act on the novel coronavirus (2019-nCoV), Wuhan, China through a drug-target interaction deep learning model. bioRxiv, Doi: 2020.01.31.929547*;* Gao et al., 2020, Machine intelligence design of 2019-nCoV drugs. bioRxiv. 2020.01.30.927889)*.* The previous computational results reported a very low binding energy from -10 to -11 kcal/mol. The scoring functions were different from one to another docking software.

In the present study, the inventors screened the molecular interaction of CID_129111 with the prominent non-polar covalent bonding with SARS-CoV-2. The inventors computationally proved that the CID_129111 had stronger interaction with the protein with different docking parameters. All the results revealed that the best-fit docking poses occurred as the first ranking based on estimation binding-free energies and the residual contacts.

### Conclusion

In summary, CID_129111 may act as a best drug target candidate for the new X-ray crystallography structure of SARS-CoV-2. The docking prediction and ranking of ligand binding poses were significant to understand the molecular inter-connections with the protein. The study could drive a central way to achieve the clinical and *in vitro* studies in the future.

### Example 2. Study of molecular interactions of high-resolution experimental structure of the main protease (Mpro) of FIPV and SARS-CoV-1 with CID-129111 using docking analysis.

### Computational Methods

### Protein and Ligand collection

The crystallography structures of FIPV main protease in complex with dual inhibitors (Mpro/3CL) (PDB ID: 5EU8_2.45Å) (Wang et al., Journal of virology, 2016, 90(4), 1910-1917*)* and of SARS-CoV-1 in complex with N-[(1 R)-2-(tert-butylamino)-2-oxo-1-(pyridin-3-yl)ethyl]-N-(4-tert-butylphenyl)furan-2-carboxamide inhibitor (PDB ID: 3V3M_1.96 Å) were obtained from the protein databank. The 3D structure of clitocine (CID_129111) was collected from PubChem database.

The FIPV Mpro [PDB ID: 5EU8_2.45Å] has an amino acid sequence as defined in SEQ ID NO: 3.

The SARS-CoV-1 Mpro [PDB ID: 3V3M_1.96 Å] has an amino acid sequence as defined in SEQ ID NO: 2.

### Molecular docking study

Molecular docking study was carried out as reported in Example 1, except that docking calculations were carried out on 5EU8 and 3V3M protein models. The inventors performed the protein-ligand interaction by increasing the binding site radius (around 20 or 25 Å to 60 Å) for the comparative prediction of ligand affinity towards the protein.

### Binding site analysis

The inventors submitted the specific chain (A) of FIPV and SARS-CoV-1 proteins to computed atlas of surface topography of protein (CASTp) server for the prediction of binding site pockets using alpha shape method.

### Substrate binding site prediction

The substrate bounded protein complex models were submitted to Discovery studio visualizer. Then the inventors removed the water from the crystallography structure and selected the ligand groups for displaying the substrate-binding site (SBD), which showed as a sphere shape around the SBD site. Furthermore, the inventors collected the attribute of the sphere to find the XYZ grid for the docking analysis.

### Docking Analysis I and II (FIPV with CID_129111)

The affinity (grid) maps of 25 x 25 x 25 Å (nx, ny, and nz) grid points and cx = -46.30, cy=-15.30, cz = -10.73 were selected as a grid box. The 0.375 Å spacing was generated using the Autogrid program (Morris, G. M., et al. J. Comput. Chem., 1998, 19(14), 1639-1662). The ligand (clitocine, CID_129111) was optimized using MMFF94 gasteiger charge calculation method and the ligand had MMFF94 energy of 28.19533 kcal/mol. All rotatable torsions were released during docking. Each docking experiment was derived from 100 different runs that were set to terminate after a maximum of 2500000 energy evaluations. The population size was set to 150. During the search, a translational step of 0.2 Å, and quaternion and torsion steps of 5 were applied. The docking analysis II was performed by the grid maps of 60 x 60 x 60 Å (nx, ny, nz) grid points and cx = -46.30, cy=-15.30, cz = -10.73 were selected as a grid box. Furthermore, the same parameters of ligand and docking run were used for the analysis.

### Docking Analysis III and IV (SARS-CoV-1 with CID_129111)

The grid maps of 20 x 20 x 20 Å (nx, ny, nz) grid points and cx = 24.44, cy = -28.68, cz = -3.94 were selected as a grid box. The 0.375 Å spacing was generated using the Autogrid program (Morris, G. M., et al. J. Comput. Chem., 1998, 19(14), 1639-1662). The docking simulation was performed for the flexible ligands into the site-specific binding pocket (radius 20 Å). The docking analysis IV was performed by the grid maps of 60 x 60 x 60 Å (nx, ny, nz) grid points and cx = 24.44, cy = -28.68, cz = -3.94 were selected as a grid box. The same parameters of ligand and docking run were used for the analysis. Furthermore, the residual interactions were analyzed using UCSF chimera, Discovery studio visualizer tool.

### Results and Discussion

### Binding site analysis

The binding pockets were predicted around 1.4 radius probe in the protein model. FIPV has several sites on the domain interface, and surface sites. The inventors focused on the substrate-binding area (119.519A²) and the volume of the pocket (63.807A³), depicted as white cartoon bubble shape **(****Figure 6****).** Additionally, the inventors tabulated the SBD residues of FIPV. SARS-CoV-1 has the similar binding pocket of SARS-CoV-2 with the residual changes.

The Mpro of FIPV (PDB: 5EU8) and SARS-CoV-1 (PDB: 3V3M) were docked to CID_129111 using Autodock tools. The inventors performed the sequence alignment and superimposition of SARS-CoV-2 and FIPV for the prediction of structural matches between proteins **(****Figure 7****).**

The inventors selected the best ligand interaction file based on hydrogen bond, docking pose and the ligand binding free energy values. The inventors obtained top hit ligand-binding poses from the different docking analyses. The inventors used two main approaches of FIPV docking analysis: one based on site-specific docking of SBD pocket and another one based on the global flexible ligand binding with the largest docking radius **(****Figure 8****).**

Regarding the structure of Mpro FIPV, it has been reported that the protein had SBD site for the accommodation of N3 complex and the dual inhibitor molecules in the bound structure. Additionally, the SBD site of FIPV was very similar to the transmissible gastroenteritis coronavirus (TGEV) SBD site in the pig. It has also been reported that the SBD sites were classified as S1, S2, S3, S4, S5 and S' subsites. The key amino acids N25, V26, L27, H41, T47, Y53, F139, C144, H162, H163, L164, E165, G167, Q187, P188, S189, M190 were constituting the SBD sites for the inhibitors. The catalytic residues H41 and C144 were present in the FIPV structure (Wang et al., Journal of virology, 2016, 90(4), 1910-1917*).*

### Docking analysis I

The CID_129111 was docked to the 5EU8A structure by site-specific interaction. In the present invention, the CID_129111 had molecular interactions with the SBD site of FIPV with several strong H-bond contacts. The 4^{th} binding pose had lowest estimation binding free energy (-7.15 kcal/mol with all the corresponding positions of S1, S2, S3, S4 in SBD site as reported previously (Wang et al., Journal of virology, 2016, 90(4), 1910-1917)*.* Furthermore, the inventors observed several H-bond (T47, E165, L166, G167, Q187, S189), Pi-Sigma (L164), Pi-Alkyl (L166) interactions **(Table 6 below).** The similar residual interaction was reported in earlier studies (Theerawatanasirikul, S., et al. Antiviral Research, 2020, 174, 104697)*.* The Pi-Sigma showed very strong contact with the ligand equivalent to the H-bond. This result confirmed that the ligand occupied the SBD site and created favorable strong contacts with the FIPV protein **(****Figure 9****).**

### Docking analysis II

The CID_129111 was docked to the 5EU8A structure with 60 Å radius. The results showed that the CID_129111 compound had several H-bond contacts (N25, V26, E118, and G142), but the ligand was able to perform the interaction at the SBD site, while the inventors carried out the docking procedure using the global flexible method. The estimation binding free energy score (-6.00 kcal/mol) was little less compared to the site-specific docking **(****Figure 10****).**

### Docking analysis III

The inventors obtained the molecular docking results of SARS-CoV-1 with CID_129111 on site-specific interaction. These results prominently depicted that CID_129111 had a very strong interaction with the SARS-CoV-1 structure **(****Figure 11****).**

The H-bond contact (F140, N142, G143, S144, C145, E166) of ligand showed that the ligand occupied well the SBD site by building the Pi-Sulfur interaction with the C145 residue and the Amide Pi-stacked interaction in L141 residue. The docking score of FIPV was computed as -6.89 kcal/mol of binding-free energy with CID_129111 **(Table 6).**

**Table 6. The residual interaction and the binding-free energy value of FIPV and SARS-CoV-1 Mpro-ligand docking.**

| **S.N o** | **Bind -ing pose** | **Receptor PDB Chain** | **Est. Free Energy of Binding** | **Est. Inhibition Constant, Ki** | **H bond** | **Hydrophobic interactions (Pi-Alkyl/Pi-Sigma/Pi-Sulfur/Amide-Pi-Stacked)** | **Grid radius (Å)** | **Frequency** | **Int. Surface** |
|---|---|---|---|---|---|---|---|---|---|
| 1 | 4 | FIPV 5EU8A | -7.15 kcal/mol | 5.71 uM | T47, E165, L166, G167, Q187, S189 | L164, L166 | 25x25 x25 | 5% | 545.296 |
| 2 | 1 | FIPV 5EU8A | -6.00 kcal/mol | 40.29 uM | N25, V26, H41, E118, G142 | | 60x60 x60 | 2% | 521.906 |
| 3 | 1 | SARS-CoV-1 3V3MA | -6.89 kcal/mol | 8.96 uM | F140, N142, G143, S144, C145, E166 | L141, C145 | 25x25 x25 | 17% | 456.859 |
| 4 | 2 | SARS-CoV-1 3V3MA | -6.35 kcal/mol | 22.19 uM | L141, N142, S144, C145 | N142, C145 | 60x60 x60 | 2% | 510.517 |

### Docking Analysis IV

The inventors obtained the molecular docking results of SARS-CoV-1 with CID_129111 on global-flexible docking analysis. The estimation binding-free energy showed -6.35 kcal/mol and the inhibition constant was of 22.19 uM in the flexible blind docking. **Figure 12** shows the H-bond residues (L141, N142, S144, C145) and hydrophobic residues (N142 and C145). These results are in accordance with the similar residual interaction that has been reported for SARS-CoV-1 studies (Jacobs et al., Journal of medicinal chemistry, 2013, 56(2), 534-546*).* Specifically, the C145 residue had the covalent bonding with CID_129111 as reported in the SARS-CoV-2 study (Ton et al., Molecular Informatics, 2020, 1-8*).*

In the prior art, several compounds have been used as a potential drug target for FIPV (Theerawatanasirikul, S., et al. Antiviral Research, 2020, 174, 104697*)* and SARS-CoV-1 (Jacobs et al., Journal of medicinal chemistry, 2013, 56(2), 534-546*).* Three molecules have also been reported as drug-like fragments (NSC87511, NSC343256, and NSC345647) for FIPV using compuatational docking study. The protein-ligand interactions observed by the inventors were corroborated with the previous reports of residual interactions. The inventors screened the molecules using important strategies of selecting the molecule based on binding poses of ligand interaction with the non-polar covalent bonding and the binding-free energies. All the best-fit docking poses were obtaining the estimation, binding-free energies and the best residual contacts.

### Conclusion

In summary, the ligand showed strong affinity towards FIPV and SARS-CoV-1. Therefore, CID_129111 may act as a best drug target candidate for the FIPV and the SARS-CoV-1 protein models. The inventors utilized the application of *in silico*-based analysis of protein-ligand interactions and the ligand screening approaches.

### Example 3. Study of molecular interactions of high-resolution experimental structure of the main protease (Mpro) of porcine transmissible gastroenteritis virus (TGEV), Porcine Epidemic Diarrhea Virus (PEDV) and Bovine Coronavirus hemagglutinin-esterase (BCoV-HE) with CID-129111 using docking analysis.

### Computational Methods

### Protein and Ligand collection

The crystallography structure of porcine transmissible gastroenteritis virus (TGEV) Mpro in complex with an inhibitor N1 (Mpro/3CL) (PDB ID: 2AMP_2.70Å) and of porcine epidemic diarrhea virus (PEDV) Mpro with GC376 (Mpro/3CL) (PDB ID: 6L70_1.56Å) were obtained from the protein databank. The crystallography structure of bovine coronavirus HE (BCoV-HE) in complex with 4,9-O-diacetyl sialic acid (PDB ID: 3CL5_1.80Å) was also obtained from the protein databank. The protein chain was selected for the docking analysis. The 3D structure of clitocine (CID_129111) was collected from PubChem database.

The TGEV Mpro [PDB ID: 2AMP_2.70Å] has an amino acid sequence as defined in SEQ ID NO: 5.

The PEDV Mpro [PDB ID: 6L70_1.56Å] has an amino acid sequence as defined in SEQ ID NO: 6.

The BCoV-HE [PDB ID: 3CL5_1.80Å] has an amino acid sequence as defined in SEQ ID NO: 7.

### Molecular docking study

Molecular docking study was carried out as reported in Example 1, except that docking calculations were carried out on 2AMP, 6L70 and 3CL5 protein models. The inventors performed the protein-ligand interaction using site-specific docking (radius around 25Å) for the prediction of ligand affinity towards the substrate-binding site of the protein.

### Binding site analysis

The inventors submitted the specific chain (A) of TGEV, PEDV and BCoV-HE proteins to computed atlas of surface topography of protein (CASTp) server for the prediction of binding site pockets using alpha shape method.

### Substrate-binding site prediction

The substrate bounded protein complex models were submitted to Discovery studio visualizer. Then the inventors removed the water from the crystallography structure and selected the ligand groups for displaying the substrate-binding site (SBD), which showed as a sphere shape around SBD site. Furthermore, the inventors collected the attribute of the sphere to find the XYZ grid for the docking analysis.

### Docking Analysis of TGEV (2AMP) with CID_129111

The affinity (grid) maps of 25 x 25 x 25 Å (nx, ny, and nz) grid points and cx = -8.403247, cy= -18.692871, cz = -15.677409 were selected as a grid box. The 0.375 Å spacing was generated using the Autogrid program (Morris, G. M., et al. J. Comput. Chem., 1998, 19(14), 1639-1662). The ligand (CID_129111) was optimized using MMFF94 gasteiger charge calculation method and the ligand had MMFF94 energy of 28.19533 kcal/mol. All rotatable torsions were released during docking. Each docking experiment was derived from 100 different runs that were set to terminate after a maximum of 2500000 energy evaluations. The population size was set to 150. During the search, a translational step of 0.2 Å, and quaternion and torsion steps of 5 were applied.

### Docking Analysis of PEDV (6L70) with CID_129111

The grid maps of 25 x 25 x 25 Å (nx, ny, nz) grid points and cx = 18.619949, cy = -13.207831, cz = -1.351508 were selected as a grid box. The 0.375 Å spacing was generated using the Autogrid program (Morris, G. M., et al. J. Comput. Chem., 1998, 19(14), 1639-1662). The docking simulation was performed for the flexible ligands into the site-specific binding pocket (radius 25 Å). Furthermore, the same parameters of ligand and docking run were used for the analysis.

### Docking Analysis of BCoV-HE (3CL5) with CID_129111

The grid maps of 25 x 25 x 25 Å (nx, ny, nz) grid points and cx = 19.349311, cy = -11.999508, cz = -15.461000 were selected as a grid box. The 0.375 Å spacing was generated using the Autogrid program (Morris, G. M., et al. J. Comput. Chem., 1998, 19(14), 1639-1662). The docking simulation was performed for the flexible ligands into the site-specific binding pocket (radius 25 Å). Furthermore, the same parameters of ligand and docking run were used for the analysis, and the residual interactions were analyzed using UCSF chimera, Discovery studio visualizer tool.

### Results and Discussion

### Binding site analysis

The binding pockets were predicted around 1.4 radius probe in the protein model. There are several sites on the domain interface and surface sites. The inventors focused on substrate binding area and the volume of the pocket **(****Figures 13, 14** **and** **15****).** Additionally, the inventors tabulated the SBD residues of the TGEV, PEDV and BCoV-HE. Few similar conserved residues were present in SARS-CoV-1 and SARS-CoV-2.

The inventors selected the best ligand interaction file based on hydrogen bonds, docking pose and the ligand binding free energy values. The inventors obtained top hit ligand-binding poses from the different docking analyses. In this study, the inventors focused mainly on the SBD site with short docking radius.

### Docking analysis of TGEV

CID_129111 was docked to the TGEV structure (2AMPA) using site-specific docking analysis. In this study, the inventors observed the molecular interactions of the ligand (CID_129111) in the SBD site of TGEV with several strong H-bond contacts. The first rank binding pose had very lowest estimation binding free energy (-7.39 kcal/mol) with all the corresponding position of S1, S1' and S2 SBD site as reported previously (Wang et al., Journal of virology, 2016, 90(4), 1910-1917*;* Jin et al., Structure of M pro from COVID-19 virus and discovery of its inhibitors, Nature, 2020*).* Additionally, the interaction site was similar to the P1, P2, P3 of new SARS-CoV-2 report (Zhang, et al. Science, 2020, 368(6489), 409-412)*.* Furthermore, the inventors observed several H-bonds (1140, G142, C144, H162), Pi-Alkyl (A141) interactions **(Table 7 below).**

The similar residual interaction was reported in the earlier studies on feline infectious peritonitis virus (FIPV) protein (Theerawatanasirikul, S., et al. Antiviral Research, 2020, 174, 104697)*.* This result confirmed that the ligand engaged to the SBD site and formed favorable strong contacts with the TGEV protein **(****Figure 16****).**

### Docking analysis of PEDV

The CID_129111 was docked to the 6L70A structure with 25Å radius. The results showed that the ligand had several strong H-bond contacts (E165, Q187, P188, T189) and Pi-Alkyl (L166, P188), Amide-Pi Stacked (P188). The ligand had the best amide bond interaction with the protein. The inventors also observed the docking pose 2 for this protein, which showed more H-bond interactions (I140, G142, C144, H162, E165) and Pi-Donor (C144). The ligand was able to perform the interaction at the SBD site and make interactions with the catalytic link with the C144 residue. An estimation binding free energy score of Pose 1= -7.13 kcal/mol and Pose 2 = -7.09 kcal/mol was obtained **(****Figure 17****).**

### Docking analysis of BCoV-HE

The inventors obtained the molecular docking results of BCoV-HE with CID_129111 on site-specific interaction. Usually the crystal structure of HE protein is complexed with O-diacetyl sialic acid. The present result showed that the ligand occupied the corresponding SBD site **(****Figure 18****).** The strong H-bond of ligand interactions (T114, L210, F211, L212, T242, T243) were observed in the SBD site. The docking score of BCoV-HE was computed as - 6.99 kcal/mol of binding-free energy with CID_129111 **(Table 7).** The ligand interaction was identified only for the conventional and carbon-hydrogen bonds.

**Table 7. The residual interaction and the binding-free energy value of FIPV and SARS-CoV Mpro-ligand docking.**

| **S.No** | **Docking Binding pose** | **Receptor_PDB**_**Chain** | **Est. Free Energy of Binding** | **Est. Inhibition Constant, Ki** | **H bond** | **Hydrophobic interactions (Pi-Alkyl/Pi-Donor/Amid e-Pi-Stacked)** | **Grid radius (Å)** | **Frequency** |
|---|---|---|---|---|---|---|---|---|
| 1 | 1 | TGEV_ 2AMPA | -7.39 kcal/mol | 3.81 uM | I140, G142, C144, H162 | A141 | 25x 25x 25 | 16% |
| 2 | 1 | PEDV_ 6L70 | -7.13 kcal/mol | 5.92 uM | E165, Q187, T189 | L166, P188 | 25x 25x 25 | 10% |
| 3 | 2 | PEDV_ 6L70 | -7.09 kcal/mol | 6.34 uM | I140, G142, C144, H162, E165 | C144 | 25x 25x 25 | 5% |
| 4 | 1 | BCoV-HE_3CL5 | -6.99 kcal/mol | 7.49 uM | T114, L210, F211, L212, T242, T243 | | 25x 25x 25 | 9% |

Several inhibitors have been used to make a crytallographic structure complex such as chloromethyl ketone (CMK) (Yang, H., et al, PLoS biology, 2005, 3(10*)),* GC376 (Ye, G., etal. Viruses, 2020, 12(2), 240)*,* O-diacetyl sialic acid and acetic acid (Zeng, Q., et al. PNAS, 2008, 105(26), 9065-9069)*.* The protein-ligand interactions observed by the inventors were in accordance with the previous reports of residual interactions. The inventors screened the molecules using important strategies of selecting the molecule based on binding poses of ligand interaction with the non-polar covalent bonding and the binding-free energies. All the best-fit interactions were obtained in the first rank of docking poses with the estimation, binding-free energies and the SBD residual contacts.

### Conclusion

In summary, the ligand showed strong affinity towards the proteins. Therefore, CID_129111 may act as a best drug target candidate for TGEV, PEDV and BCoV-HE protein models. The inventors utilised the novel computational application of the protein-ligand interactions to screen the binding affinity of ligands.

## Claims

1. A composition comprising (i) a compound selected from the group consisting of clitocine, a pharmacophore for clitocine, tautomer, mesomer, racemate, enantiomer, diastereomer, or mixture thereof, or an acceptable salt thereof, and (ii) a pharmaceutically acceptable vehicle, a carrier, an excipient or a diluent, for use in the prevention and/or the treatment of an infection by a virus from the *Coronaviridae* family or an illness related to such infection, in a host, in particular a mammalian host.

2. The composition of claim 1 for use according to claim 1, wherein said viral infection or illness is selected from the group consisting of SARS, COVID-19, FIP, TGE, PED, and enteric and respiratory disease.

3. The composition of claim 1 or 2 for use according to claim 1 or 2, wherein the virus from the *Coronaviridae* family is selected from the group consisting of SARS-CoV-1, SARS-CoV-2, FIPV, TGEV, PEDV and BCoV.

4. The composition of any one of claims 1 to 3 for use according to any one of claims 1 to 3, comprising clitocine.

5. The composition of any one of claims 1 to 4 for use according to any one of claims 1 to 4, wherein the compound is selected from the group consisting of compounds 2-90, preferably is selected from the group consisting of compounds 2-76, even more preferably is selected from the group consisting of compounds 2-10, 11 and 77-90.

6. The composition of claim 5 for use according to any one of claims 1 to 5, wherein the compound is selected from the group consisting of compounds 2-10.

7. The composition of any one of claims 1 to 6 for use according to any one of claims 1 to 6, wherein the mammalian host is a human host.

8. The composition of any one of claims 1 to 7 for use according to any one of claims 1 to 7, wherein the mammalian host is selected from the group consisting of a pig, a bovine animal, a horse, a cat, a dog, a rabbit, a rodent, a bird and a bat, preferably is a pig, a bovine animal or a cat.

9. The composition of any one of claims 1 to 8 for use in association with another therapeutic agent, in particular an antibiotic, in the prevention and/or the treatment of an infection by a virus from the *Coronaviridae* family or an illness related to such infection according to any one of claims 1 to 3.


SEQUENCE LISTING

<110>  INSTITUT DE RECHERCHE EN SEMIOCHIMIE ET ETHOLOGIE APPLIQUEE

<120>  NUCLEOSIDE ANALOGUES TO INHIBIT THE MAIN PROTEASE OF A
       CORONAVIRUS

<130>  B14297/AD/DP/KN

<140>  EPxxxxx
<141>  2020-05-20

<160>  7

<170>  PatentIn version 3.5

<210>  1
<211>  306
<212>  PRT
<213>  Artificial sequence

<220>
<223>  Main protease of SARS-CoV-2 (PDB: 6LU7, 2.16 A°)

<400>  1

<210>  2
<211>  306
<212>  PRT
<213>  Artificial sequence

<220>
<223>  Main protease of SARS-CoV-1 (PDB: 3V3M)

<400>  2

Ser Gly Phe Arg Lys Met Ala Phe Pro Ser Gly Lys Val Glu Gly Cys
1               5                   10                  15

Met Val Gln Val Thr Cys Gly Thr Thr Thr Leu Asn Gly Leu Trp Leu
            20                  25                  30

Asp Asp Thr Val Tyr Cys Pro Arg His Val Ile Cys Thr Ala Glu Asp
        35                  40                  45

Met Leu Asn Pro Asn Tyr Glu Asp Leu Leu Ile Arg Lys Ser Asn His
    50                  55                  60

Ser Phe Leu Val Gln Ala Gly Asn Val Gln Leu Arg Val Ile Gly His
65                  70                  75                  80

Ser Met Gln Asn Cys Leu Leu Arg Leu Lys Val Asp Thr Ser Asn Pro
                85                  90                  95

Lys Thr Pro Lys Tyr Lys Phe Val Arg Ile Gln Pro Gly Gln Thr Phe
            100                 105                 110

Ser Val Leu Ala Cys Tyr Asn Gly Ser Pro Ser Gly Val Tyr Gln Cys
        115                 120                 125

Ala Met Arg Pro Asn His Thr Ile Lys Gly Ser Phe Leu Asn Gly Ser
    130                 135                 140

Cys Gly Ser Val Gly Phe Asn Ile Asp Tyr Asp Cys Val Ser Phe Cys
145                 150                 155                 160

Tyr Met His His Met Glu Leu Pro Thr Gly Val His Ala Gly Thr Asp
                165                 170                 175

Leu Glu Gly Lys Phe Tyr Gly Pro Phe Val Asp Arg Gln Thr Ala Gln
            180                 185                 190

Ala Ala Gly Thr Asp Thr Thr Ile Thr Leu Asn Val Leu Ala Trp Leu
        195                 200                 205

Tyr Ala Ala Val Ile Asn Gly Asp Arg Trp Phe Leu Asn Arg Phe Thr
    210                 215                 220

Thr Thr Leu Asn Asp Phe Asn Leu Val Ala Met Lys Tyr Asn Tyr Glu
225                 230                 235                 240

Pro Leu Thr Gln Asp His Val Asp Ile Leu Gly Pro Leu Ser Ala Gln
                245                 250                 255

Thr Gly Ile Ala Val Leu Asp Met Cys Ala Ala Leu Lys Glu Leu Leu
            260                 265                 270

Gln Asn Gly Met Asn Gly Arg Thr Ile Leu Gly Ser Thr Ile Leu Glu
        275                 280                 285

Asp Glu Phe Thr Pro Phe Asp Val Val Arg Gln Cys Ser Gly Val Thr

290                 295                 300

Phe Gln
305

<210>  3
<211>  307
<212>  PRT
<213>  Artificial sequence

<220>
<223>  Main protease of FIPV (PDB: 5EU8)

<400>  3

Gly Pro Leu Gly Ser Ser Gly Leu Arg Lys Met Ala Gln Pro Ser Gly
1               5                   10                  15

Val Val Glu Pro Cys Ile Val Arg Val Ala Tyr Gly Asn Asn Val Leu
            20                  25                  30

Asn Gly Leu Trp Leu Gly Asp Glu Val Ile Cys Pro Arg His Val Ile
        35                  40                  45

Ala Ser Asp Thr Ser Arg Val Ile Asn Tyr Glu Asn Glu Leu Ser Ser
    50                  55                  60

Val Arg Leu His Asn Phe Ser Ile Ala Lys Asn Asn Ala Phe Leu Gly
65                  70                  75                  80

Val Val Ser Ala Lys Tyr Lys Gly Val Asn Leu Val Leu Lys Val Asn
                85                  90                  95

Gln Val Asn Pro Asn Thr Pro Glu His Lys Phe Lys Ser Val Arg Pro
            100                 105                 110

Gly Glu Ser Phe Asn Ile Leu Ala Cys Tyr Glu Gly Cys Pro Gly Ser
        115                 120                 125

Val Tyr Gly Val Asn Met Arg Ser Gln Gly Thr Ile Lys Gly Ser Phe
    130                 135                 140

Ile Ala Gly Thr Cys Gly Ser Val Gly Tyr Val Leu Glu Asn Gly Thr
145                 150                 155                 160

Leu Tyr Phe Val Tyr Met His His Leu Glu Leu Gly Asn Gly Ser His
                165                 170                 175

Val Gly Ser Asn Leu Glu Gly Glu Met Tyr Gly Gly Tyr Glu Asp Gln
            180                 185                 190

Pro Ser Met Gln Leu Glu Gly Thr Asn Val Met Ser Ser Asp Asn Val
        195                 200                 205

Val Ala Phe Leu Tyr Ala Ala Leu Ile Asn Gly Glu Arg Trp Phe Val
    210                 215                 220

Thr Asn Thr Ser Met Thr Leu Glu Ser Tyr Asn Ala Trp Ala Lys Thr
225                 230                 235                 240

Asn Ser Phe Thr Glu Ile Val Ser Thr Asp Ala Phe Asn Met Leu Ala
                245                 250                 255

Ala Lys Thr Gly Tyr Ser Val Glu Lys Leu Leu Glu Cys Ile Val Arg
            260                 265                 270

Leu Asn Lys Gly Phe Gly Gly Arg Thr Ile Leu Ser Tyr Gly Ser Leu
        275                 280                 285

Cys Asp Glu Phe Thr Pro Thr Glu Val Ile Arg Gln Met Tyr Gly Val
    290                 295                 300

Asn Leu Gln
305

<210> 4
<211> 306
<212> PRT
<213> Artificial sequence

<220>
<223> Main protease of SARS-CoV-2 (PDB: 7BQY, 1.7 A°)

<400> 4

<210>  5
<211>  304

<212>  PRT
<213>  Artificial sequence

<220>
<223>  Main protease of porcine TGEV (PDB: 2AMP)

<400>  5

Gly Ser Ser Gly Leu Arg Lys Met Ala Gln Pro Ser Gly Leu Val Glu
1               5                   10                  15

Pro Cys Ile Val Arg Val Ser Tyr Gly Asn Asn Val Leu Asn Gly Leu
            20                  25                  30

Trp Leu Gly Asp Glu Val Ile Cys Pro Arg His Val Ile Ala Ser Asp
        35                  40                  45

Thr Thr Arg Val Ile Asn Tyr Glu Asn Glu Met Ser Ser Val Arg Leu
    50                  55                  60

His Asn Phe Ser Val Ser Lys Asn Asn Val Phe Leu Gly Val Val Ser
65                  70                  75                  80

Ala Arg Tyr Lys Gly Val Asn Leu Val Leu Lys Val Asn Gln Val Asn
                85                  90                  95

Pro Asn Thr Pro Glu His Lys Phe Lys Ser Ile Lys Ala Gly Glu Ser
            100                 105                 110

Phe Asn Ile Leu Ala Cys Tyr Glu Gly Cys Pro Gly Ser Val Tyr Gly
        115                 120                 125

Val Asn Met Arg Ser Gln Gly Thr Ile Lys Gly Ser Phe Ile Ala Gly
    130                 135                 140

Thr Cys Gly Ser Val Gly Tyr Val Leu Glu Asn Gly Ile Leu Tyr Phe
145                 150                 155                 160

Val Tyr Met His His Leu Glu Leu Gly Asn Gly Ser His Val Gly Ser
                165                 170                 175

Asn Phe Glu Gly Glu Met Tyr Gly Gly Tyr Glu Asp Gln Pro Ser Met
            180                 185                 190

Gln Leu Glu Gly Thr Asn Val Met Ser Ser Asp Asn Val Val Ala Phe
        195                 200                 205

Leu Tyr Ala Ala Leu Ile Asn Gly Glu Arg Trp Phe Val Thr Asn Thr
    210                 215                 220

Ser Met Ser Leu Glu Ser Tyr Asn Thr Trp Ala Lys Thr Asn Ser Phe
225                 230                 235                 240

Thr Glu Leu Ser Ser Thr Asp Ala Phe Ser Met Leu Ala Ala Lys Thr
                245                 250                 255

Gly Gln Ser Val Glu Lys Leu Leu Asp Ser Ile Val Arg Leu Asn Lys
            260                 265                 270

Gly Phe Gly Gly Arg Thr Ile Leu Ser Tyr Gly Ser Leu Cys Asp Glu
        275                 280                 285

Phe Thr Pro Thr Glu Val Ile Arg Gln Met Tyr Gly Val Asn Leu Gln
    290                 295                 300

<210>  6
<211>  305
<212>  PRT
<213>  Artificial sequence

<220>
<223>  Main protease of PEDV (PDB: 6L70)

<400>  6

Ala Gly Leu Arg Lys Met Ala Gln Pro Ser Gly Val Val Glu Lys Cys
1               5                   10                  15

Ile Val Arg Val Cys Tyr Gly Asn Met Ala Leu Asn Gly Leu Trp Leu
            20                  25                  30

Gly Asp Thr Val Met Cys Pro Arg His Val Ile Ala Ser Ser Thr Thr
        35                  40                  45

Ser Thr Ile Asp Tyr Asp Tyr Ala Leu Ser Val Leu Arg Leu His Asn
    50                  55                  60

Phe Ser Ile Ser Ser Gly Asn Val Phe Leu Gly Val Val Gly Val Thr
65                  70                  75                  80

Met Arg Gly Ala Leu Leu Gln Ile Lys Val Asn Gln Asn Asn Val His
                85                  90                  95

Thr Pro Lys Tyr Thr Tyr Arg Thr Val Arg Pro Gly Glu Ser Phe Asn
            100                 105                 110

Ile Leu Ala Cys Tyr Asp Gly Ala Ala Ala Gly Val Tyr Gly Val Asn
        115                 120                 125

Met Arg Ser Asn Tyr Thr Ile Arg Gly Ser Phe Ile Asn Gly Ala Cys
    130                 135                 140

Gly Ser Pro Gly Tyr Asn Ile Asn Asn Gly Thr Val Glu Phe Cys Tyr
145                 150                 155                 160

Leu His Gln Leu Glu Leu Gly Ser Gly Cys His Val Gly Ser Asp Leu
                165                 170                 175

Asp Gly Val Met Tyr Gly Gly Tyr Glu Asp Gln Pro Thr Leu Gln Val
            180                 185                 190

Glu Gly Ala Ser Ser Leu Phe Thr Glu Asn Val Leu Ala Phe Leu Tyr
        195                 200                 205

Ala Ala Leu Ile Asn Gly Ser Thr Trp Trp Leu Ser Ser Ser Arg Ile
    210                 215                 220

Ala Val Asp Arg Phe Asn Glu Trp Ala Val His Asn Gly Met Thr Thr
225                 230                 235                 240

Val Val Asn Thr Asp Cys Phe Ser Ile Leu Ala Ala Lys Thr Gly Val
                245                 250                 255

Asp Val Gln Arg Leu Leu Ala Ser Ile Gln Ser Leu His Lys Asn Phe
            260                 265                 270

Gly Gly Lys Gln Ile Leu Gly Tyr Thr Ser Leu Thr Asp Glu Phe Thr
        275                 280                 285

Thr Gly Glu Val Ile Arg Gln Met Tyr Gly Val His His His His His
    290                 295                 300

His
305

<210>  7
<211>  377
<212>  PRT
<213>  Artificial sequence

<220>
<223>  BCoV-HE (PDB: 3CL5)

<400>  7

Phe Asp Asn Pro Pro Thr Asn Val Val Ser His Leu Asn Gly Asp Trp
1               5                   10                  15

Phe Leu Phe Gly Asp Ala Arg Ser Asp Cys Asn His Val Val Asn Thr

20                  25                  30

Asn Pro Arg Asn Tyr Ser Tyr Met Asp Leu Asn Pro Ala Leu Cys Asp
        35                  40                  45

Ser Gly Lys Ile Ser Ser Lys Ala Gly Asn Ser Ile Phe Arg Ser Phe
    50                  55                  60

His Phe Thr Asp Phe Tyr Asn Tyr Thr Gly Glu Gly Gln Gln Ile Ile
65                  70                  75                  80

Phe Tyr Glu Gly Val Asn Phe Thr Pro Tyr His Ala Phe Lys Cys Thr
                85                  90                  95

Thr Ser Gly Ser Asn Asp Ile Trp Met Gln Asn Lys Gly Leu Phe Tyr
            100                 105                 110

Thr Gln Val Tyr Lys Asn Met Ala Val Tyr Arg Ser Leu Thr Phe Val
        115                 120                 125

Asn Val Pro Tyr Val Tyr Asn Gly Ser Ala Gln Ser Thr Ala Leu Cys
    130                 135                 140

Lys Ser Gly Ser Leu Val Leu Asn Asn Pro Ala Tyr Ile Ala Arg Glu
145                 150                 155                 160

Ala Asn Phe Gly Asp Tyr Tyr Tyr Lys Val Glu Ala Asp Phe Tyr Leu
                165                 170                 175

Ser Gly Cys Asp Glu Tyr Ile Val Pro Leu Cys Ile Phe Asn Gly Lys
            180                 185                 190

Phe Leu Ser Asn Thr Lys Tyr Tyr Asp Asp Ser Gln Tyr Tyr Phe Asn
        195                 200                 205

Lys Asp Thr Gly Val Ile Tyr Gly Leu Asn Ser Thr Glu Thr Ile Thr
    210                 215                 220

Thr Gly Phe Asp Phe Asn Cys His Tyr Leu Val Leu Pro Ser Gly Asn
225                 230                 235                 240

Tyr Leu Ala Ile Ser Asn Glu Leu Leu Leu Thr Val Pro Thr Lys Ala
                245                 250                 255

Ile Cys Leu Asn Lys Arg Lys Asp Phe Thr Pro Val Gln Val Val Asp
            260                 265                 270

Ser Arg Trp Asn Asn Ala Arg Gln Ser Asp Asn Met Thr Ala Val Ala
        275                 280                 285

Cys Gln Pro Pro Tyr Cys Tyr Phe Arg Asn Ser Thr Thr Asn Tyr Val
    290                 295                 300

Gly Val Tyr Asp Ile Asn His Gly Asp Ala Gly Phe Thr Ser Ile Leu
305                 310                 315                 320

Ser Gly Leu Leu Tyr Asp Ser Pro Cys Phe Ser Gln Gln Gly Val Phe
                325                 330                 335

Arg Tyr Asp Asn Val Ser Ser Val Trp Pro Leu Tyr Ser Tyr Gly Arg
            340                 345                 350

Cys Pro Thr Ala Ala Asp Ile Asn Thr Pro Asp Val Pro Ile Cys Val
        355                 360                 365

Tyr Asp Ser Asp Pro Leu Val Pro Arg
    370                 375